# EUROPEAN PATENT SPECIFICATION

(11) **EP 0 450 515 B1**
(45) Date of publication and mention of the grant of the patent: **12.07.1995**
(21) Application number: 91105003.7
(22) Date of filing: 28.03.1991
(51) Int. Cl.: C07J 1/00, C07J 7/00, C07J 5/00, A61K 31/56, C07J 41/00, C07J 51/00, C07J 21/00, C07J 17/00

(54) **Haloethyl-substituted steroidal enzyme inhibitors**
Haloethyl substituierte enzymhemmende Steroiden
Enzyme inhibitant, steroides, substitués par haloéthyle

(30) Priority: 02.04.1990 US 503191
(43) Date of publication of application: 09.10.1991
(73) Proprietor: MERRELL DOW PHARMACEUTICALS INC., Cincinnati Ohio 45215 (US)
(72) Inventor: Burkhart, Joseph P., West Chester, Ohio 45069 (US); Peet, Norton P., Cincinnati, Ohio 45241 (US); Johnston, J. O'Neal, Milford, Ohio 45150 (US)
(74) Representative: VOSSIUS & PARTNER

(56) References cited:
- EP-A- 0 227 472
- GB-A- 1 037 162
- GB-A- 2 185 258
- US-A- 3 213 085
- STEROIDS, STRUCTURE, FUNCTION AND REGULATION, vol. 50, nos 4-6, 1987, pages363-374; D.F. COVEY et al.: "Metabolism of 19-methyl substituted steroids andaproposal for the third aromatase monooxygenation"
- BIOCHEMISTRY, vol. 26, no. 24, 1st December 1987, pages 7833-7841; D.D.BEUSENet al.: "Metabolism of 19-methyl-substituted steroids by humanplacentalaromatase"
- STEROIDS, vol. 45, nos 3,4, March-April 1985, pages 357-374; J.P. BURKHARTetal.: "Novel silylated steroids as aromatase inhibitors"
- STEROIDS, vol. 45, nos 3,4, March-April 1985, pages 297-302; K.H. SCHÖNEMANNetal.: "Structure and progestational activiy of 13-substituted-18-norpregn-4-ene-3,20-diones, a pilot study"
- P.A.MARCOTTE et al. Steroids Vol.39, 325 (1982)

## Description

The estrogen hormones, estrone and estradiol, which are involved in many physiological processes, are formed from cholesterol via several enzymatic steps. The enzyme aromatase is the final rate limiting enzyme in the nonreversible conversion of the androgen hormones, testosterone and androstenedione, to the estrogen hormones, estradiol and estrone. Compounds such as aromatase inhibitors may thus regulate or inhibit androgen to estrogen conversion, and have therapeutic utility in treating clinical conditions potentiated by the presence of estrogens.

19-Nordeoxycorticosterone (19-norDOC) is known to induce mineralocorticoid hypertension. In the biosynthetic formation of 19-norsteroids, such as 19-norDOC, the initial step is the adrenal C₁₉ hydroxylation of an appropriate steroid such as deoxycorticosterone (DOC). The inhibition of the biosynthetic formation of 19-norDOC by inhibition of 19-hydroxylation of DOC would thus serve to decrease the level of 19-norDOC present in the animal involved and reduce hypertensive effects attributable to the presence of this material.

Aldosterone is a steroidal hormone which is synthesized in the zona glomerulosa cells of the adrenal glands. The primary biological function of the compound is the regulation of salt retention. In particular, aldosterone plays a major role in controlling the reabsorption of sodium ions from the kidney filtrates. Thus, a deficiency of the enzyme responsible for the synthesis of aldosterone is a characteristic of patients with a salt-losing syndrome, while primary hyperaldosteronism can result from hyperbiosynthesis of aldosterone as caused by an adrenocortical tumor or the administration of certain drugs. The hyperaldosteronism may involve hypertension, hypokalemia, alkalosis, muscular weakness, polyuria, and polydipsia. Thus, treatment of hyperaldosteronism and the conditions associated with it would be possible by blockage of the enzymatic synthesis of aldosterone.

The present invention is directed to novel haloethyl-substituted steroidal enzyme inhibitors, their related intermediates, and processes for their preparation. These compounds may be represented by the following formulas: 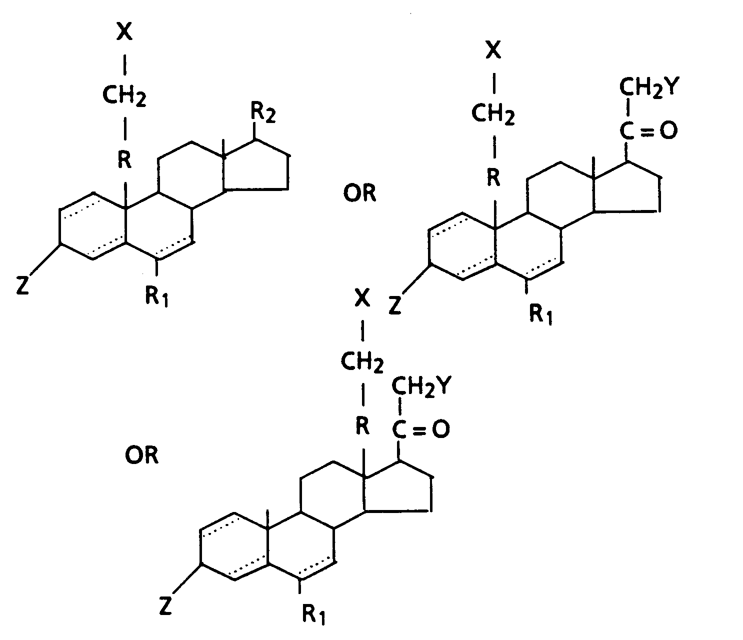
wherein
---- represents a single or double bond,
X = Br, Cl, or I,
R = CHOH or C=O,
R₁ = H, C₁₋₄ alkyl, =O, or -OH,
R₂ = =O, -OH, or -O-(C₁₋₄ alkanoyl),
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

Examples of C₁₋₄ alkyl include methyl, ethyl, propyl, isopropyl, butyl, and isobutyl. Examples of C₁₋₄ alkanoyl include formyl, acetyl, propionyl, and butyryl. When R is CHOH, two optical isomers are possible. The present invention encompasses the individual pure isomers, or mixtures of the two isomers in any proportion. The (R) isomer for the halohydrin moiety at C₁₀ is preferred for aromatase activity.

The compounds of the present invention are inhibitors of aromatase, 19-hydroxylase, and aldosterone biosynthesis. As aromatase inhibitors, they are useful in treating hyperestrogenemia. The compounds are useful in controlling abnormally high levels of estrogens, both when the high levels observed are relatively steady, or when there are brief surges of elevated levels occurring as part of cyclical body functions. Both females and males can be treated, although obviously, the level of estrogen which would be considered high in males would be much lower than the amount considered high in females. These compounds are also useful as anti-fertility agents to prevent ovulation or implantation in females, or to reduce the mating behavior in males where brain aromatization is required for such behavior. These compounds further have value in treating gynecomastia, male infertility resulting from elevated estrogen levels, and hyperestrogenemia, which may proceed myocardial infarction. The compounds may also have value in the treatment of breast cancer and various estrogen-induced or estrogen-stimulated disorders, such as benign prostatic hypertrophy and endometrial hyperplasia.

The bioconversion of deoxycorticosterone via a 19-hydroxylase pathway to 19-nordeoxycorticosterone potentiates its mineralocorticoid activity. Mineralocorticoid excess results in a syndrome characterized by hypokalemia, metabolic alkalosis, polydipsia, polyuria, and hypertensive conditions. Increased excretion of 19-nordeoxycorticosterone has been reported for hypertensive patients, including those with primary aldosteronism, Cushing's syndrome, 17α-hydroxylase deficiency, and individuals with essential hypertension. As 19-hydroxylase inhibitors, these compounds may be useful as antihypertensive agents and for management of edematous conditions often associated with sodium retention and potassium loss.

As inhibitors of aldosterone, these compounds are useful for the treatment of hyperaldosteronism and various conditions wherein a reduction of the excess amount of aldosterone responsible for the condition would be beneficial. Thus, they are useful in the treatment of hyperaldosteronism and any associated hypertension, edema, and sodium retention, whether this is a result of some bodily disorder, or whether it results from the administration of some agent. As a result of their effects on the factors responsible for edema or sodium retention, the indicated compounds would be useful as diuretic agents.

To achieve their desired effect, the compounds of the present invention may be administered orally, parenterally, for example, intravenously, intraperitoneally, intramuscularly, or subcutaneously, including the injection of the active ingredient directly into tissue or tumor sites, to a patient in need of treatment. The term patient is taken to mean a warm-blooded animal, for example, mammals such as humans, primates, cattle, dogs, cats, horses, sheep, mice, rats, and pigs. These compounds may also be administered in the form of a pharmaceutical preparation, and may further be incorporated into sustained delivery devices. The amount of compound administered will vary over a wide range and be any effective amount. Depending on the patient to be treated, the condition to be treated, and mode of administration, the effective amount of compound administered will vary from about 0.01 to 150 mg/kg of body weight per day, and preferably from about 0.1 to 50 mg/kg body weight per day.

For oral administration, the compounds can be formulated into solid or liquid preparations, such as capsules, pills, tablets, troches, powders, solutions, suspensions, or emulsions. The solid unit dosage forms can be a capsule which can be of the ordinary gelatin type containing the active compound and a carrier, for example, lubricants and inert fillers such a lactose, sucrose and corn starch. In another embodiment, an active compound of the invention can be tableted with conventional tablet bases such as lactose, sucrose and corn starch in combination with binders such as acacia, corn starch, or gelatin, disintegrating agents such as potato starch, or alginic acids and a lubricant such as stearic acid or magnesium stearate.

For parenteral administration the compounds may be administered as injectable dosages of a solution or suspension of the compound in a physiological acceptable diluent with a pharmaceutical carrier which can be a sterile liquid such as water-in-oil with or without the addition of a surfactant and other pharmaceutically acceptable adjuvants. Illustrative of oils which can be employed in these preparations are those of petroleum, animal, vegetable or synthetic origin, for example, peanut oil, soybean oil, and mineral oil. In general, water, saline, aqueous dextrose and related sugar solutions, ethanol and glycols, such as propylene glycol or polyethylene glycol are preferred liquid carriers, particularly for injectable solutions.

The compounds can be administered in the form of a cutaneous patch, a depot injection, or implant preparation which can be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers and synthetic silicones, for example, Silastic®, silicone rubber manufactured by Dow Corning Corporation. Further information on suitable pharmaceutical carriers and formulation techniques are found in standard texts such as Remington's Pharmaceutical Sciences, Mack Publishing Company, Easton, Pennsylvania.

Inhibition of aromatase activity is demonstrated by using laboratory methods similar to procedures described in U.S. Patent No. 4,322,416, and as published in Johnston et al., Endocrinology 115:776, 1984, and Burkhart et al., Steroids 45:357, 1985.

In this assay, the inhibitor is preincubated with enzyme prior to assaying for activity in the presence of high substrate levels. A time-related decrease in enzyme activity can be indicative of irreversible binding of the inhibitor with the enzyme.

In the time-dependent assay, an amount of the enzyme inhibitor in 100 »l of the assay buffer described above which will provide assay concentrations which are usually between 1 nM and 10 »M are added to 35 ml centrifuge tubes containing 600 »l of the NADPH generating system. The pre-incubation is started by the addition of 700 »l of aromatase preparation, usually 50-800 »g of microsomal protein per ml of assay buffer. These preparations are mixed using a vortex mixer and incubated for 0, 5, 10, or 20 minutes at 25°C. Then 100 »l of androstenedione (∼6.8 »M) containing 1β-³H androstenedione in assay buffer is added to provide an assay concentration of substrate (0.50 »M) which is at least ten times the Kₘ of androstenedione (0.04 »M). Following vortexing, the enzyme incubation is continued for 10 minutes before being terminated by the addition of chloroform. The amount of radioactivity in the aqueous fraction is determined by scintillation procedures. The enzymatic activity for each concentration of inhibitor at each time period of preincubation is calculated as a percent of the respective vehicle control which is arbitrarily set at 100%. Therefore, the relative present enzyme inhibition is expressed as a percentage: (100 percent minus percent enzyme activity with inhibitor present).

Enzyme kinetic analysis utilized Kitz-Wilson plots for time-dependent assays. These analyses provide estimates of apparent Kᵢ of inactivation which represents the inhibitor concentration required to produce half-maximal rate of enzyme inactivation. The pseudo first-order rate constant for enzyme inactivation (k_{cat}) and the half-time of inactivation (τ₅₀) of infinite inhibitor concentrations were determined. The ratio of k_{cat}/Kᵢ (inactivation) provides an index number which increases with increased efficiency of enzyme inactivation and increased inhibitor affinity for the enzyme active site.

The compounds listed below exhibited the following results

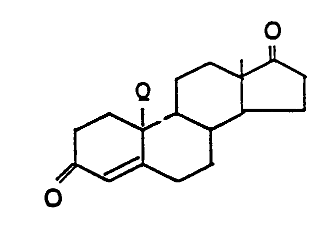

| Q | Kᵢ(nM) | τ50(min) | k_{cat}/Kᵢ |
|---|---|---|---|
| HOCHCH₂Br (S):(R)::9:1 | 1134 | 5.33 | 1,900 |
| HOCHCH₂Br pure (R) diastereomer | 26 | 4.85 | 92,100 |
| O=CCH₂Br | 190 | 32.7 | 1,860 |
| HOCHCH₂Cl (R):(S)::9:1 | 63 | 3.60 | 50,700 |
| HOCHCH₂I (R):(S)::9:1 | 11 | 2.22 | 490,000 |

Compounds to be assayed for 1β-19-hydroxylase inhibiting activity are solubilized in dimethylsulfoxide (DMSO) at a concentration of 10 mM and diluted with assay buffer (10 mM KCl, 1 mM EDTA, 100 mM Tris at pH 8.0) to provide the necessary concentrations. The assays are conducted in 35 ml glass tubes maintained at 25°C in a Dubnoff shaker with 95% 0₂/5% CO₂ atmosphere. The assay tubes contain the following: 100 »l of an NADPH-generating system (5 mM NADP, 15 mM glucose-6-phosphate, and 5 I.U./ml glucose-6-phosphate dehydrogenase), 300 »l of hamster adrenal mitochondrial protein, 50 »l of test compound or buffer (control), and 50 »l tritium-labeled substrate, i.e., 1 »M [³H]DOC.

Compounds are evaluated for their inhibition by preincubating with the enzyme preparation supplemented with the NADPH-generating system for 0 to 60 min at 25°C prior to the addition of radiolabeled substrate. Assays are incubated for varying time intervals from 1 to 60 min. Assays are quenched by the addition of 5 ml of ethyl acetate. Nonradiolabeled steroids are added and samples are extracted twice with 5 ml of ethyl acetate, and the solvent is evaporated under nitrogen at 30-40°C.

The residues are redissolved in 10 mM Na acetate:acetonitrile 1:1 v/v (pH 6.0), and high performance liquid chromatography (HPLC) is used to separate products on two C₁₈ Radial Pak columns (Waters, Millipore Corporation, Milford, MA) in series (5 »m particles, 0.8x10 cm each). Chromatographic buffer A is 10% CH₃CN/90% 19 mM Na acetate (pH 6.0), and buffer B is 80% CH₃CN/20% 10 mM Na acetate (pH 6.0). The amount of remaining labeled DOC substrate and initial hydroxylated products, corticosterone and 19-hydroxy-DOC, are separated and the radioactivity contained in each peak is quantified. The 19-hydroxylase activity is based on the quantity of radiolabeled DOC metabolized, since hamster adrenal corticosterone and 19-hydroxy-DOC are the products of a single enzyme.

Unlabeled steroids are monitored by their absorbance at 240 nm with an inline spectrometer. The extinction coefficients for derivatives of DOC are assumed to be similar to that of DOC (ε = 17,200 M⁻¹cm⁻¹). Radioactivity of DOC metabolites is measured using an inline scintillation spectrometer with a 1 ml flow cell.

Time-dependent enzyme inhibition is evaluated by pre-incubating the enzyme with steroidal compound for either 0 or 60 minutes at 25°C prior to the addition of radiolabeled substrate for a 5 minute assay. The apparent Kₘ for the initial hydroxylation of DOC may be estimated by the double reciprocal plot of Lineweaver-Burk. IC₅₀'s may be graphically estimated from linear-log plots of enzyme activities and log of inhibitor concentrations.

The activity of the present compounds as aldosterone biosynthesis inhibitors can be demonstrated by the following procedure which measures the inhibition of enzymes in the synthesis of aldosterone.

Young male Sprague-Dawley rats are maintained on a sodium-deficient diet for about two weeks prior to use. From these animals, adrenal capsule/glomerulose homogenates are prepared (6 mg/ml) in pH 7.4 assay buffer [MgCl₂ 8.5 mM, CaCl₂ 2.7 mM, KCl 3.13 mM, NaCl 7.591 mM, TRIS 50 mM, and 0.1% triethylamine] and centrifuged 500xg for 10 minutes.

Assays are conducted in 35 ml glass tubes maintained at 25°C in a Dubnoff shaker with 95% O₂/5% CO₂. The tubes contain the following material: 100 »l of a NADPH generating system, 300 »l of adrenal capsular/glomerulosa cytosol, and 50 »l of test compound or buffer (control). After initial incubation intervals of 20 minutes, the 10 minute assay is started by the addition of 50 »l tritium-labelled substrate, i.e., 1 »M [³H]-DOC. Reactions are quenched by the addition of 5 ml of ethyl acetate and nonradiolabelled steroids are also added. The samples are extracted twice with 5 ml of ethyl acetate and the solvent is evaporated under nitrogen at 30-40°C.

Residues are redissolved in methanol:water (40:60) with 0.1% triethylamine and high performance liquid chromatography is used to separate products on a C18 reverse phase (5 » ODS-Hypersil) column (4.6 x 250 mm, Shannon) with a 1 ml/min flow rate using an MeOH:H₂O gradient (solvent A 10/90:solvent B 90/10). Unchanged substrate and products formed are monitered by UV absorbance at 246 nM and the amount of tritiated steroid compounds present is quantified by radioactivity measurement.

The preparation of these compounds may be illustrated by the following scheme: 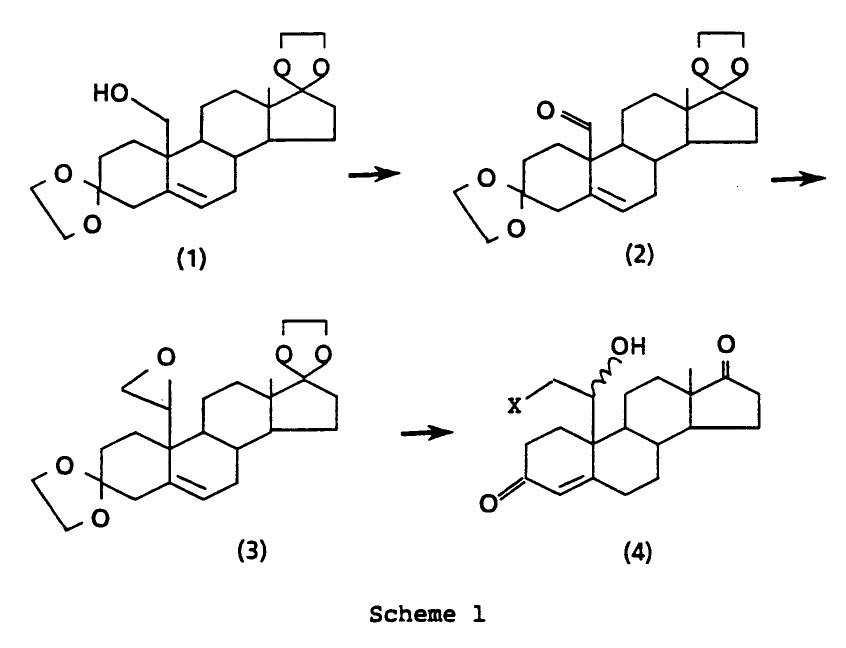
The known steroidal alcohol, 3,3:17,17-bis[1,2-ethanediylbis(oxy)]-androst-5-en-19-ol (1) is reacted with dimethyl sulfoxide and oxalyl chloride in CH₂Cl₂. Et₃N is added to the resulting mixture. This mixture is treated with CH₂Cl₂/H₂O and the layers separated. Flash chromatography of the organic layer yields the desired steroidal aldehyde compound (2).

The steroidal aldehyde (2) is treated with sodium dimsylate and trimethylsulfonium iodide. This mixture is then added to a mixture of Et₂O/H₂O and the layers are separated. The organic layer yields a mixture of diastereomers of the steroidal epoxide (3). To an acetone solution of the steroidal epoxide (3) is added aqueous halo-acid (HX, wherein X = Br, Cl, or I), then CH₂Cl₂. The organic layer yields upon flash-chromatography the halo-alcohol (4).

To prepare the corresponding 1,4-diene compound, for example, 10-(2-bromo-1-hydroxyethyl)-estr-1,4-diene-3,17-dione, a catalytic amount of acid, such as p-toluenesulfonic acid, is added to an aqueous acetone solution of the steroidal epoxide (3). The resulting mixture is added to a mixture of EtOAc/NaHCO₃. Flash chromatography of the organic layer yields the steroidal epoxide 4-ene-dione as a mixture of diastereomers. This product is reacted with 2,3-dichloro-5,6-dicyano-1,4-benzoquinone in dioxane, with heating, to give the corresponding epoxide 1,4-diene-dione. This product is then reacted with hydrobromic acid in acetone to give 10-(2-bromo-1-hydroxyethyl)-estr-1,4-diene-3,17-dione.

To prepare the corresponding 4,6-diene compound, for example, 10-(2-bromo-1-hydroxyethyl)-estr-4,6-diene-3,17-dione, the corresponding epoxide 4-ene-dione is reacted with tetra-chloro-1,4-benzoquinone in toluene to give the corresponding epoxide 4,6-diene-dione. This product is then reacted with hydrobromic acid in acetone to give 10-(2-bromo-1-hydroxyethyl)-estr-4,6-diene-3,17-dione.

To prepare the 1,4,6-triene compound, for example, 10-(2-bromo-1-hydroxyethyl)-estr-1,4,6-triene-3,17-dione, the corresponding epoxide 4,6-diene-dione is reacted with 2,3-dichloro-5,6-dicyano-1,4-benzoquinone in dioxane with heating to yield the corresponding epoxide 1,4,6-triene-dione. This product is then reacted with hydrobromic acid in acetone to give 10-(2-bromo-1-hydroxyethyl)-estr-1,4,6-triene-3,17-dione.

The halo-ketone (5) may be prepared by the oxidation of alcohol (4) according to Scheme 2, below: 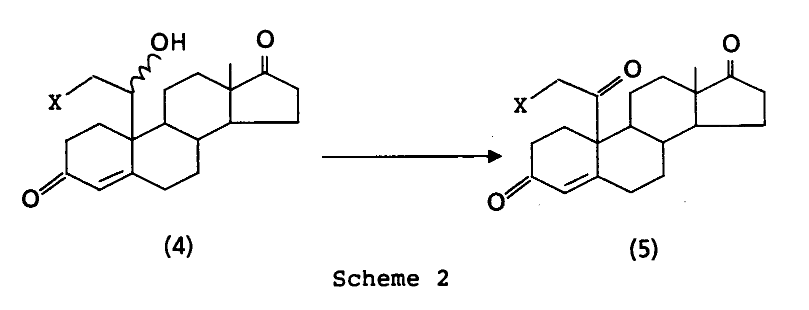
To a solution of the halo-alcohol (4) in acetone is added dropwise Jones reagent (CrO₃/H₂SO₄/H₂O). The reaction is quenched with isopropanol, diluted with CH₂Cl₂/H₂O, and the layers separated. Chromatography yields the halo-ketone (5).

To prepare compounds having the hydroxyacetyl substituent at the 17-position, Scheme 3 is utilized: 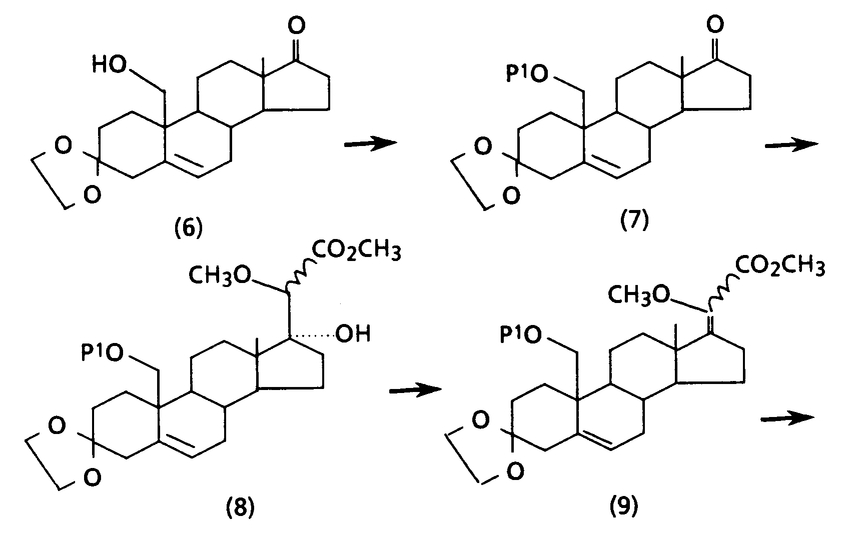 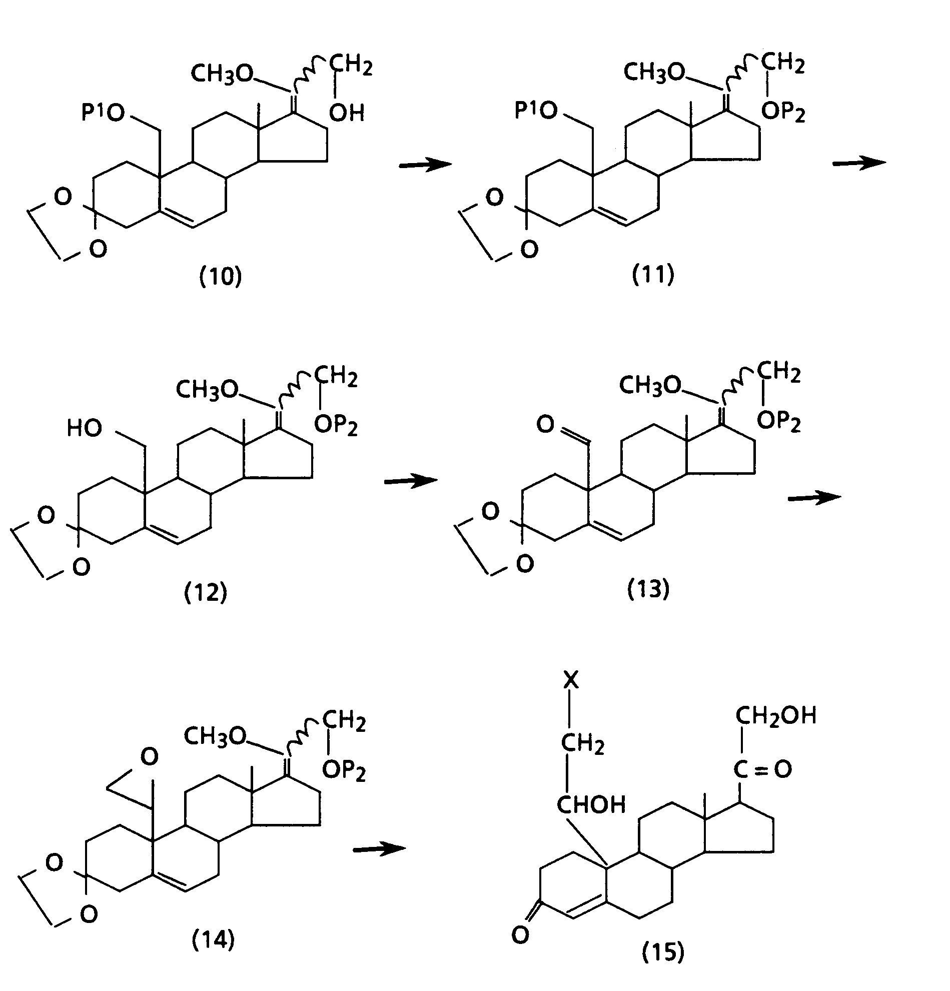
wherein 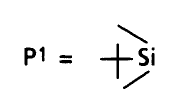
and 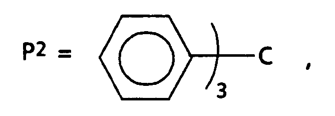
or 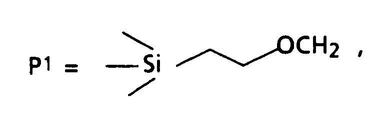
and P² = CH₃OCH₂, and
X = Br, Cl, or I.

Specifically, the 21-hydroxypregnane compounds of the present invention can be prepared from the appropriate 17-keto steroid. Thus, for example, 3,3-ethylenedioxy-19-hydroxyandrost-5-en-17-one (6) is reacted with (2-chloromethoxyethyl)trimethylsilane and diisopropylethylamine in methylene chloride to give the corresponding compound (7) in which the 19-alcohol is protected by a 2-(trimethylsilyl) ethoxymethyl group. This compound is then reacted with methyl methoxyacetate and lithium diisopropylamide whereupon the indicated ester (i.e., the methylene group thereof), adds across the 17-ketone to give the 17-substituted 17-hydroxy steroid (8). Dehydration using thionyl chloride and pyridine introduces a 17-exocyclic double bond and the resulting α-methoxy ester (9) is reduced with DIBAL to the corresponding 20-methoxy alcohol (10) which is then further treated with chloromethyl methyl ether and diisopropyl amine in methylene chloride to protect the hydroxy group as the methoxymethyl ether (11). The silyl group protecting the 19-alcohol is then selectively removed by treatment with tetra(n-butyl)ammonium fluoride in tetrahydrofuran to give the 19-hydroxy compound (12). The 19-hydroxy group is then oxidized to the corresponding aldehyde (13) using a standard Swern oxidation. Reaction of the aldehyde with trimethylsulfonium iodide in dimethylsulfoxide gives the corresponding oxirane (14). Reaction of the oxirane with aqueous hydrohalic acid, hydrobromic acid, in acetone opens the oxirane ring to give the corresponding halohydrin, e.g. bromohydrin. At the same time the oxirane ring is opened, the acid used also serves to remove the protecting groups located elsewhere on the molecule. That is, the enol ether and the methoxymethyl ether that are part of the 17-substituent in the steroid are removed and the 17-hydroxyacetyl group results. In addition, the 3,3-ethylene-dioxy group is removed and the 3-keto-Δ⁴ compound (15) results.

To prepare those compounds wherein R₂ is -OH, the following scheme may be utilized: 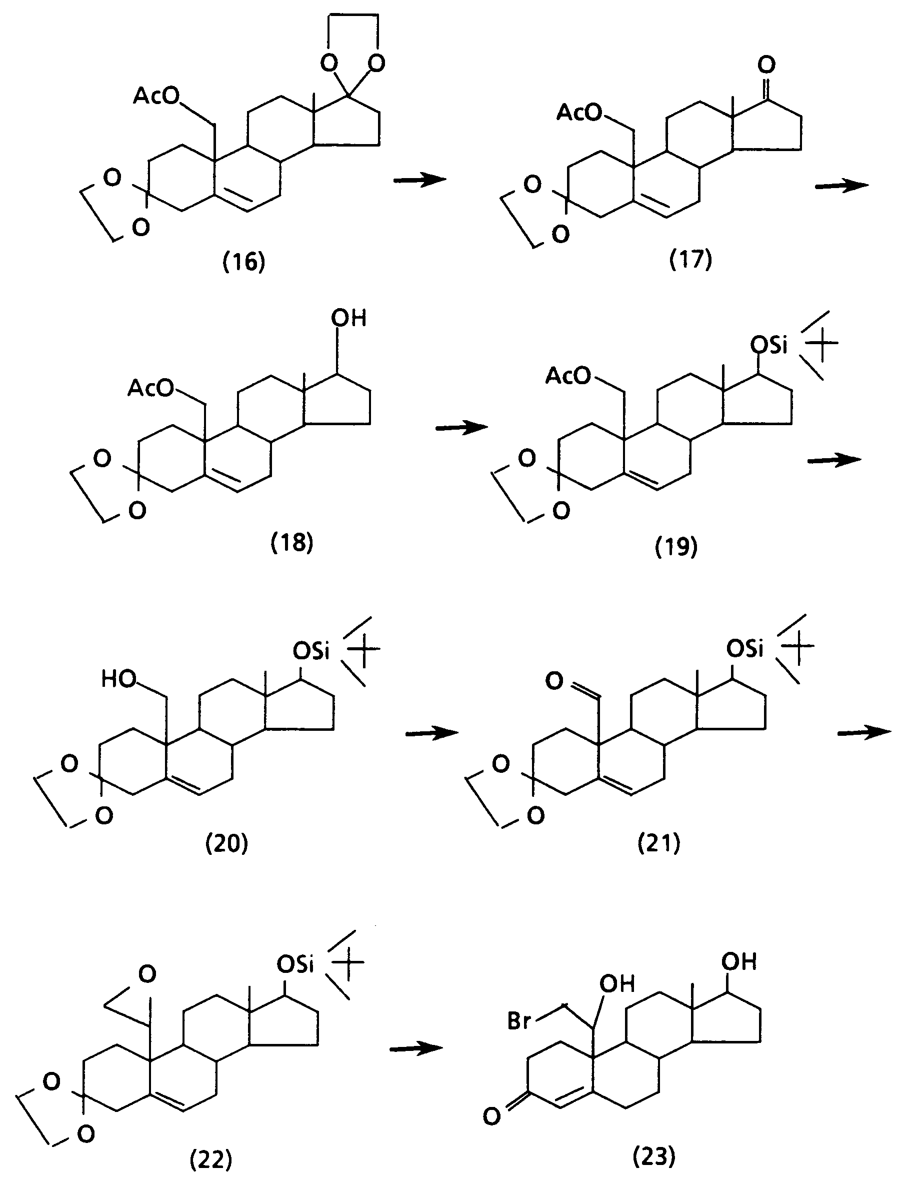
The starting compound, 19-acetoxy-3,3,17,17-bis(ethylenedioxy)androst-5-ene (16) is selectively hydrolyzed using 0.15% perchloric acid in t-butanol and methylene chloride to remove the ketal group at the 17-position and give the corresponding 17-ketone (17). The ketone function is then reduced using sodium borohydride in ethanol to give the corresponding 17β-hydroxy compound (18). The 17-hydroxy compound is then reacted with t-butyldimethylsilyl chloride in an inert solvent such as dimethylformamide in the presence of 4-dimethylaminopyridine and triethylamine to give the corresponding 17-(t-butyldimethylsilyloxy) compound (19). The 19-acetoxy group is then removed by reaction of the compound with aqueous lithium hydroxide in methanol and tetrahydrofuran to give 17β-(t-butyldimethylsilyloxy)-3,3-ethylenedioxyandrost-5-en-19-ol (20). The 19-ol is then oxidized to the corresponding aldehyde (21) using dimethyl sulfoxide and oxalyl chloride in methylene chloride followed by a tertiary amine such as triethylamine. The aldehyde is then reacted with trimethylsulfonium iodide in dimethylsulfoxide to give the corresponding oxirane (22). The oxirane is then converted to the desired corresponding bromohydrin (23) using hydrobromic acid in acetone. The conditions used to open the oxirane ring also serve to remove the ketal protecting group at the 3-position and the silyl ether group at the 17-position to give 10-(2-bromo-1-hydroxyethyl)-17β-hydroxyestr-4-en-3-one (23).

The preparation of the 18-halohydrin compounds may be illustrated by the following scheme: 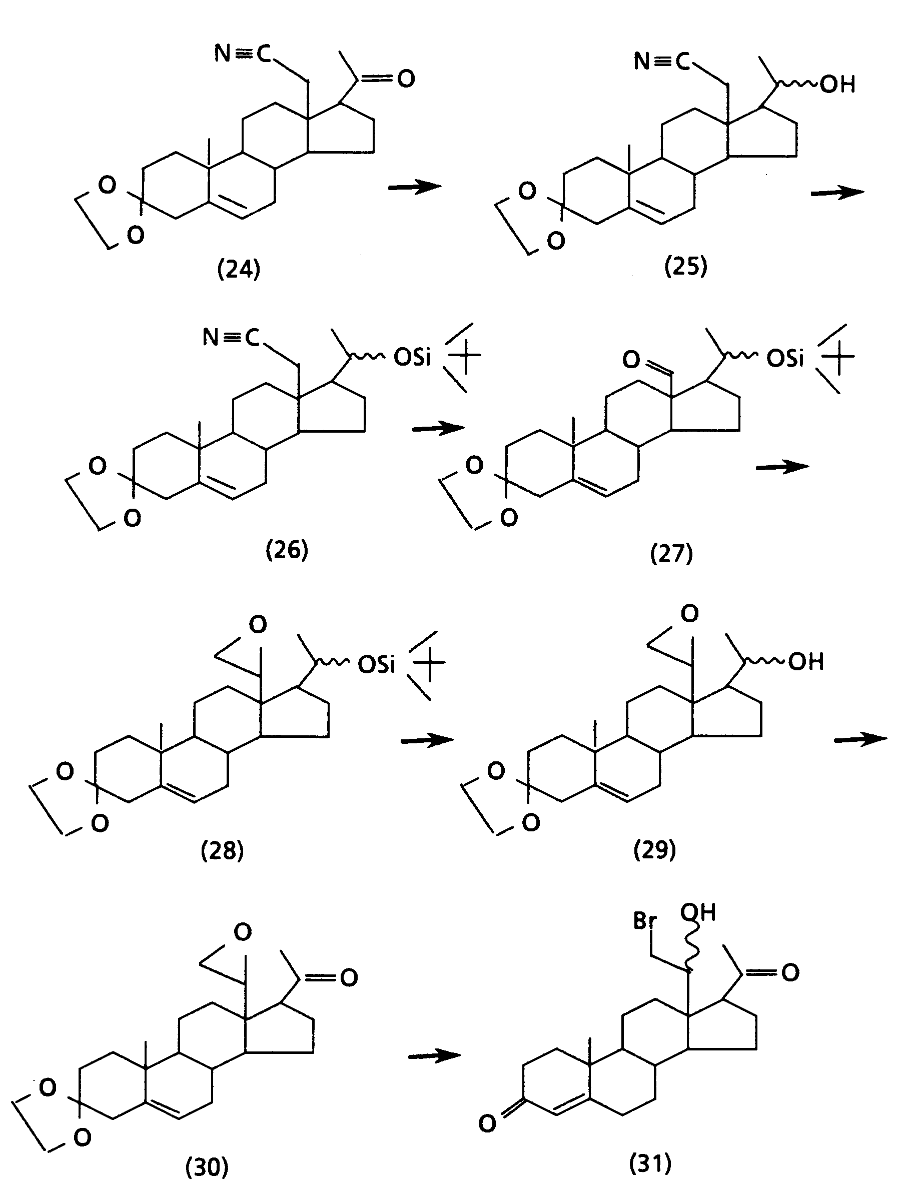
The compound 18-cyanopregn-5-ene-3,20-dione-3-ethylene ketal (24) [Freerksen et al., J. Am. Chem. Soc., 99, 1536 (1977)] is reacted with sodium borohydride to reduce the 20-ketone and give a mixture of the two epimeric 20-hydroxy compounds (25). This mixture of alcohols is converted to a mixture of the corresponding (t-butyl)dimethylsilyl ethers (26) by reaction of the alcohols with (t-butyl)dimethylsilyl chloride, 4-dimethylaminopyridine, and triethylamine in dimethylformamide. The resulting 18-cyano silyl ether is then reacted with a strong base, such as lithium diisopropyl amide, in tetrahydrofuran and hexamethylphosphoramide, followed by oxidation and then reaction with a trialkylphosphite, such as trimethyl or triethyl phosphite to give the corresponding 13-carboxaldehyde, i.e., 3,3-ethylenedioxy-20-(t-butyldimethylsilyloxy)pregn-5-en-18-al (27). The aldehyde is then reacted with trimethylsulfonium iodide, sodium dimethyl sulfoxide in tetrahydrofuran, and dimethyl sulfoxide to give the corresponding 13-oxiranyl compound (28). The silyl protecting group is then removed by treatment of the silyl ether with tetrabutylammonium fluoride to give the free 20-hydroxy compound (29) which is then subjected to a Swern oxidation to give the corresponding 20-ketone (30). The oxirane is then converted to 18-bromomethyl-18-hydroxypregn-4-ene-3,20-dione by reaction with trimethylsilyl bromide followed by dilute acid to give the bromohydrin (31). Alternatively, the oxirane (30) can be reacted with 48% hydrobromic acid in acetone to form the bromohydrin and remove the 3-ketal protecting group simultaneously, and give the desired product (31) directly. The corresponding chlorohydrin or iodohydrin may be prepared by reacting the oxirane with hydrochloric acid or hydroiodic acid, respectively.

The foregoing syntheses are illustrative, and many other conventional reactions may be used to produce or to interconvert the compounds of the invention. These conventional reactions and conditions may be found, e.g., in Fieser et al., "Steroids" (Reinhold, New York, 1959); Djerassi, Ed., "Steroid Reactions" (Holden-Day, San Francisco, 1963); Kirk et al., "Steroid Reaction Mechanisms" (Elsevier, Amsterdam, 1968); Carruthers, "Some Modern Methods of Organic Synthesis" (Cambridge U. Press, Cambridge, 1971); and Harrison et al., "Compendium of Organic Synthetic Methods" (Wiley-Interscience, New York, 1971).

The following examples are presented to illustrate the invention. They are not intended to limit the invention in any manner.

### EXAMPLE 1

### 3,3:17,17-Bis[1,2-ethanediylbis(oxy)]-androst-5-en-19-al (2)

To a stirred solution of oxalyl chloride (0.43 ml, 4.88 mmol) in CH₂Cl₂ (13 ml) under an argon atmosphere and cooled to -55°C was slowly added DMSO (0.69 ml, 9.75 mmol) diluted with CH₂Cl₂ (2 ml). After 4 minutes, 3,3:17,17-bis[1,2-ethanediylbis(oxy)]-androst-5-en-19-ol (1) (1.27 g, 3.25 mmol) in CH₂Cl₂ (5 ml) and DMSO (0.5 ml) was slowly added. The resulting suspension was stirred at -55°C for 35 minutes, Et₃N (2.72 ml, 19.50 mmol) was added, the mixture was further stirred 5 minutes, and then allowed to warm to room temperature. The mixture was poured into CH₂Cl₂ (50 ml)/H₂O (50 ml). The layers were separated and the aqueous layer was further extracted with CH₂Cl₂ (20 ml). The combined organic layers were washed with 0.5 N HCl (15 ml), saturated NaHCO₃ (25 ml), then brine (25 ml). Drying (MgSO₄) and concentration gave a tan solid which was dissolved in CH₂Cl₂ (2 ml) and loaded onto a column. Flash chromatography, using EtOAc-hexane (35:65) as the eluant, gave the desired aldehyde, 3,3:17,17-bis[1,2-ethanediylbis(oxy)]-androst-5-en-19-al (2) (1.07 g, 85% yield) as a white solid. (Melting point = 168-170°C).
Analysis (C₂₃H₃₂O₅): Calculated: C, 71.11; H, 8.30. Found: C, 71.21; H, 8.40.
¹H-NMR: (CDCl₃): δ 9.69 (s, 1H, CHO), 5.81-5.87 (m, 1H, vinyl H), 3.78-4.02 (m, 8H, 2x OCH₂CH₂O), 0.79 (m, 3H, 18-CH₃).
MS: (EI) m/z (rel. intensity): 388 (M⁺, 2), 360 (4), 359 (3), 298 (18), 297 (22), 253 (8), 235 (7), 99 (100).
(CI/CH₄) m/z (rel. intensity): 389 (MH⁺, 100), 361 (13), 327 (20), 299 (9), 99(11).

### EXAMPLE 2

### 10β-[(R)-Oxiranyl] and 10β-[(S)-Oxiranyl] compounds (3)

A stirred solution of sodium dimsylate (27 ml, 1.52 M, 41.11 mmol) under an argon atmosphere at room temperature was diluted with THF (80 ml), then cooled in an ice-salt bath. A solution of trimethylsulfonium iodide (8.39 g, 41.11 mmol) in DMSO (32 ml) was slowly added. After 10 minutes, a solution of the compound of Example 1 (3.55 g, 9.14 mmol) in THF (35 ml) was further added. After cooling 1 hour in an ice-salt bath, the cooling bath was removed and the mixture was allowed to warm to room temperature. After 75 minutes at room temperature, the mixture was poured into Et₂O (850 ml)/H₂O (350 ml). The layers were separated and the aqueous layer was further extracted with Et₂O (100 ml). The combined organic layers were washed with H₂O (2 x 300 ml) followed by brine (150 ml). Drying (MgSO₄) and concentration gave an oily foam. Crystallization from Et₂O-hexane gave the 10β-[(R)-oxiranyl] and 10β-[(S)-oxiranyl] compound (1.12 g, mixture of diastereomers; ratio 19R:19S::9:1). The filtrate was flash chromatographed, eluting with EtOAc/hexane (3:7), to give additional compound (3) (1.62 g, mixture of diastereomers; ratio 19R:19S::9:1).
Analysis (C₂₃H₃₄O₅): Calculated: C, 71.61; H, 8.71. Found: C, 71.67; E, 8.71.
¹H-NMR: (CDCl₃) δ 5.56-5.61 and 5.49-5.56 (pr m, 1H, vinyl H), 3.80-4.00 (m 8H, 2 x OCH₂CH₂O), 3.04 and 2.95 (d and t, 1H, OCH), 2.52-2.78 (m, 3H), 0.94 and 0.86 (pr s, 3H, 18-CH₃).
MS: (EI) m/z (rel. intensity): 402 (M⁺, 27), 358 (3), 99 (100).
(CI/CH₄) m/z (rel. intensity): 403 (MH⁺, 100), 402(M⁺,20), 401 (27), 385 (32), 373 (45), 341 (57), 323 (18), 311 (20), 99 (30).

### EXAMPLE 3

### 10-(2-Bromo-1-hydroxyethyl)-estr-4-ene-3,17-dione (4)

To a stirred solution of the compound of Example 2 (165 mg, 0.41 mmole) in 3 ml of acetone was added 0.5 ml of 48% aqueous hydrobromic acid. After 30 minutes, the reaction was diluted to 25 ml with H₂O and poured into CH₂Cl₂ (35 ml)/H₂O (25 ml). The layers were separated and the aqueous layer was extracted with additional CH₂Cl₂ (15 ml). The combined organic layers were washed with H₂O (35 ml) followed by brine (20 ml). Drying (MgSO₄) and concentration gave the crude bromohydrin, 10-(2-bromo-1-hydroxyethyl)-estr-4-ene-3,17-dione (4) as an oily, white, waxy solid. This product was dissolved in CH₂Cl₂ (1 ml), loaded onto a 2 x 12 cm silica gel column for flash chromatography, eluted with EtOAc/hexane (40/60), and 15 ml fractions collected. Fractions 8-14 were combined and concentrated to a white, waxy solid (131 mg). An ¹H-NMR spectrum was obtained and revealed the bromohydrin as a mixture of both diastereomers with a ratio of approximately 6:1. The resulting solid of fractions 8-14 was triturated with several mls of Et₂O, scraped from the sides of the flask, and filtered to yield the product (103 mg).
¹H-NMR: (CDCl₃) δ 5.97 and 5.93 (pr s, 1H, vinyl H), 4.38 and 4.08-4.16 (dt and m, 1H, CHO), 3.81 and 3.50 and 3.44 and 3.41 (four dd, 2H, CH₂Br), 2.59 and 2.57 (pr d, 1H, OH), 0.97 and 0.96 (pr s, 3H, 18-CH₃). Ratio 19R:19S::9:1.

### EXAMPLE 4

### 10-(2-Chloro-1-hydroxyethyl)-estr-4-ene-3,17-dione (4)

To a stirred solution of the compound of Example 2 (0.25 g, 0.62 mmole) in 5 ml of acetone was added 1 ml of 37% aqueous hydrochloric acid solution. After 30 minutes, the reaction was diluted to 25 ml with H₂O and transferred to a separatory funnel containing CH₂Cl₂ (50 ml)/H₂O (40 ml). The layers were separated and the aqueous layer was extracted with additional CH₂Cl₂ (15 ml). The combined organic layers were washed with H₂O (40 ml) followed by brine (20 ml). Drying and concentration gave crude chlorohydrin, 10-(2-chloro-1-hydroxyethyl)-estr-4-ene-3,17-dione (4) as a waxy, white solid (0.20 g). This product was dissolved in CH₂Cl₂ (1 ml) and loaded onto a 2 x 12 cm silica gel column for flash chromatography, eluted with EtOAc/hexane (50:50), and 15 ml fractions collected. Fractions 5-7 were combined and concentrated to a waxy, white solid (163 mg). To this product was added 5 ml of Et₂O/hexane (2:1) with scraping of the sides of the flask, and the resulting white solid was then filtered and dried under high vacuum over refluxing acetone. There was slight discoloration from white solid to tan solid during heating, so heating was discontinued. The sample was then dried an additional 2 hours under high vacuum without heat.
Analysis (C₂₀H₂₇ClO₃): Calculated: C, 68.46; H, 7.76. Found: C, 68.27; H, 7.94.
¹H-NMR: (CDCl₃) δ 5.97 and 5.92 (pr s, 1H, vinyl H), 4.33 and 4.09 (pr dt, 1H, CHO), 3.90 and 3.47-3.61 (dd and m, 2H, CH₂Cl), 2.64 and 2.62 (pr d, 1H, OH), 0.97 and 0.96 (pr s, 3H, 18-CH₃). Ratio 19R:19S::9:1.
MS: CI/CH₄) m/z (rel. intensity): 353(20), 352(17), 351(100), 315(21), 273(54).
IR: (KBr) 3456, 2956, 2932, 2882, 2854, 1738, 1664, 1616, 746, 692 cm⁻¹.

### EXAMPLE 5

### 10-(2-Iodo-1-hydroxyethyl)-estr-4-ene-3,17-dione (4)

To a stirred solution of the compound of Example 2 (0.25 g, 0.62 mmole) in 5 ml of acetone was added 1 ml of 50% aqueous hydroiodic acid solution. After 20 minutes, the reaction was diluted to 25 ml with H₂O and transfered to a separatory funnel containing CH₂Cl₂ (35 ml)/H₂O (60 ml). The layers were separated and the aqueous layer extracted with additional CH₂Cl₂ (2x10 ml). The combined organic layers were washed with 10% aqueous Na₂S₂O₃ (25 ml) followed by brine (20 ml). Drying (MgSO₄) and concentration gave crude iodohydrin, 10-(2-iodo-1-hydroxyethyl)-estr-4-ene-3,17-dione (4) as an orange oil. To this product was added Et₂O and the mixture was concentrated to give a yellow solid (0.26 g). This product was dissolved in CH₂Cl₂ (1 ml) and loaded onto a 2.5 x 12 cm silica gel column for flash chromatography, eluted with EtOAc/hexane (50:50), and 15 to 20 ml fractions were collected. Fractions 6-10 were combined and concentrated to give a white solid (220 mg). To this product was added 4.5 ml of Et₂O/hexane (2:1) with scraping of the sides of the flask, and the resulting white solid was filtered (171 mg) and dried under high vacuum for 4 hours (166 mg).
Analysis (C₂₀H₂₇IO₃): Calculated: C, 54.31; H, 6.15. Found: C, 54.41; H, 6.25.
¹H-NMR: (CDCl₃) δ 5.97 and 5.93 (pr s, 1H, vinyl H), 4.40 and 4.13 (pr ddd, 1H, CHO), 3.66 and 3.20-3.40 (dd and m, 2H, CH₂I), 0.97 and 0.96 (pr s, 3H, 18-CH₃). Ratio 19R:19S::9:1.
MS: (CI/CH₄) m/z (rel. intensity): 443(32), 317(20), 315(30), 273(100).
IR: (KBr): 3452, 2938, 2880, 2852, 1736, 1662, 1616, 668, 638 cm⁻¹.

### EXAMPLE 6

### 10-(2-Bromoacetyl)-estr-4-ene-3,17-dione (5)

To a stirred solution of the compound of Example 3 (45 mg, 0.11 mmole) in 6 ml of acetone was added Jones reagent (CrO₃/H₂SO₄/H₂O) dropwise until a reddish-brown color persisted for several minutes in the supernatant. Excess Jones reagent was quenched by the addition of isopropyl alcohol and the reaction was diluted with CH₂Cl₂ (35 ml)/H₂O (50 ml). The layers were separated and the aqueous layer was extracted with additional CH₂Cl₂ (15 ml). The combined organic layers were washed with H₂O (20 ml) followed by brine (15) ml). Drying (MgSO₄) and concentration gave crude 10-(2-bromoacetyl)-estr-4-ene-3,17,dione (5) as a pale yellow oil. This product was dissolved in CH₂Cl₂ (1 ml) and loaded onto a 2 x 8 cm silica gel column for flash chromatography, eluted with EtOAc/hexane (50:50), and 15 ml fractions were collected. Fractions 5-8 were collected and concentrated to give (5) as a white foam (36 mg).
HRMS: MH⁺ calculated for C₂₀H₂₅BrO₃ = 393.1065. MH⁺ found = 393.1045. Error = -5.0 ppm.
¹H-NMR: (CDCl₃) δ 6.06 (s, 1H, vinyl H), 4.19 and 4.07 (pr d, 2H, CH₂Br), 0.99 (s, 3H, 18-CH₃).
MS: (CI/CH₄) m/z (rel. intensity): 395(97), 393(97), 377(13), 375(13), 343(12), 315(100), 297(16), 273(25), 272(13), 271(22).
IR: (KBr): 2940, 2856, 1736, 1674 cm⁻¹.

## Claims (Claims for the following Contracting State(s): AT, BE, CH, DE, DK, FR, GB, IT, LI, LU, NL, SE)

1. A compound of the formula: 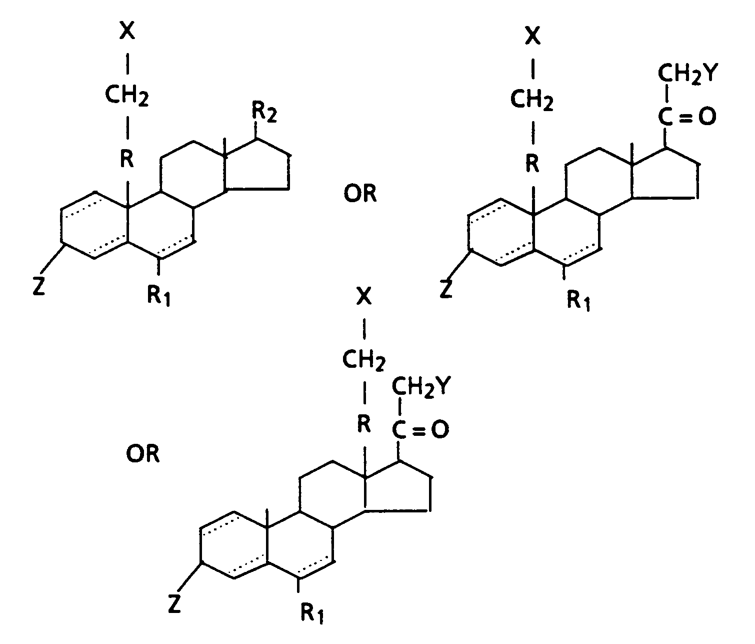 wherein
---- represents a single or double bond,
X = Br, Cl, or I,
R = CHOH or C=O,
R₁ = H, C₁₋₄ alkyl, =O, or -OH,
R₂ = =O, -OH, or -O-(C₁₋₄ alkanoyl),
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

2. A compound according to Claim 1 which has the formula 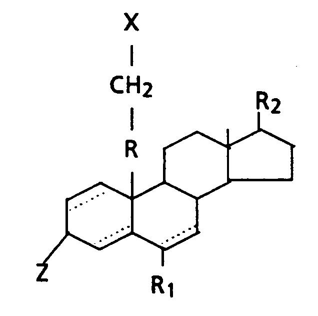 wherein
---- represents a single or double bond,
X = Br, Cl, or I,
R = CHOH,
R₁ = H, C₁₋₄ alkyl, =O, or -OH,
R₂ = =O, -OH, or -O-(C₁₋₄ alkanoyl), and
Z = =O, = CH₂, -OH, or -O-(C₁₋₄ alkanoyl)

3. A compound according to Claim 2 which has the formula 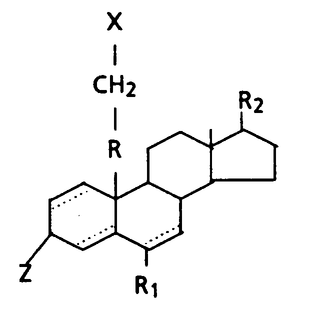 wherein
---- represents a single or double bond,
X = Br, Cl, or I,
R = CHOH,
R₁ = H or C₁₋₄ alkyl,
R₂ = =O or -OH, and
Z = =O, -OH, or -O-(C₁₋₄ alkanoyl).

4. A compound according to Claim 3 which has the formula 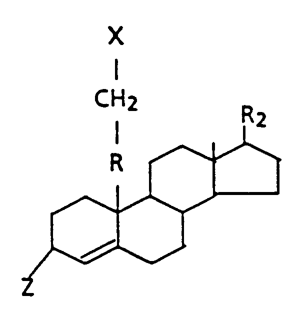 wherein
X = Br, Cl, or I,
R = CHOH,
R₂ = =O or -OH, and
Z = =O.

5. A compound according to Claim 4 which is 10-(2-bromo-1-hydroxyethyl)-estr-4-ene-3,17-dione.

6. A compound according to Claim 4 which is 10-(2-chloro-1-hydroxyethyl)-estr-4-ene-3,17-dione.

7. A compound according to Claim 4 which is 10-(2-iodo-1-hydroxyethyl)-estr-4-ene-3,17-dione.

8. A compound according to Claim 1 which is 10-(2-bromoacetyl)-estr-4-ene-3,17-dione.

9. A compound according to Claim 1 which has the formula 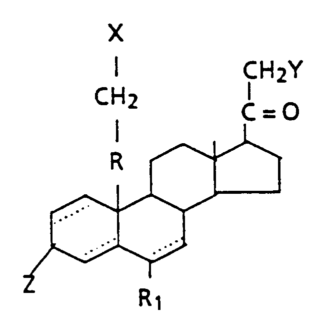 wherein
---- represents a single or double bond,
X = Br, Cl, or I,
R = CHOH or C=O,
R₁ = H, C₁₋₄ alkyl, =O, or -OH,
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

10. A compound according to Claim 9 which has the formula 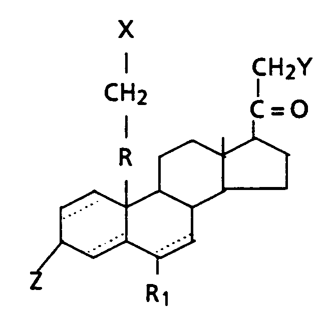 wherein
---- represents a single or double bond,
X = Br, Cl, or I,
R = CHOH,
R₁ = H or C₁₋₄ alkyl,
Z = =O, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH.

11. A compound of Claim 10 which has the formula 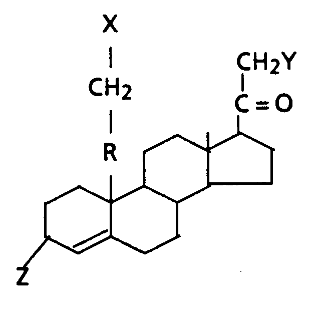 wherein
X = Br, Cl, or I,
R = CHOH,
Z = =0, and
Y = H, -OH, or -O-(C₁₋₄ alkanoyl).

12. A compound of Claim 1 which has the formula 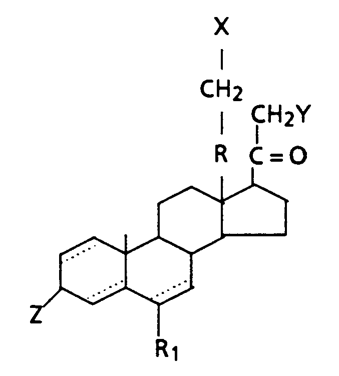 wherein
---- represents a single or double bond,
X = Br, Cl, or I,
R = CHOH or C=O,
R₁ = H, C₁₋₄ alkyl, =O, or -OH,
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

13. A compound according to Claim 12 which has the formula 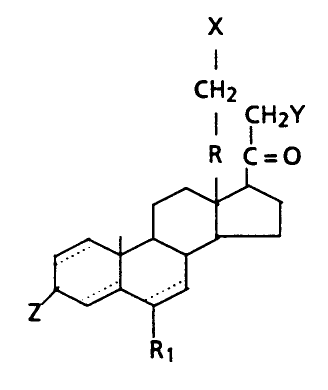 wherein
---- represents a single or double bond,
X = Br, Cl, or I,
R = CHOH,
R₁ = H or C₁₋₄ alkyl,
Z = =O, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH.

14. A compound of Claim 13 which has the formula 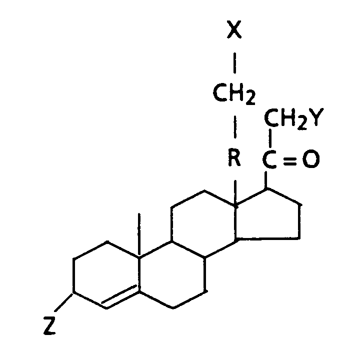 wherein
X = Br, Cl, or I,
R = CHOH,
Z = =O, and
Y = H, -OH, or -O-(C₁₋₄ alkanoyl).

15. A compound according to any one of claims 1 to 14 for use in a method of inhibiting aromatase activity, which comprises contacting an effective aromatase-inhibiting amount of the compound with an aromatase enzyme.

16. A compound according to any one of claims 1 to 14 for use in a method of treating hyperestrogenemia.

17. A compound according to any one of claims 1 to 14 for use in a method of treating estrogen-induced or estrogen-stimulated disorders.

18. A compound according to any one of claims 1 to 14 for use in a method in which the aromatase inhibitor produces an anti-fertility effect.

19. A compound according to any one of claims 1 to 14 for use in a method of treating hypertensive or edemous conditions.

20. A compound according to any one of claims 1 to 14 for use in a method for treating hyperaldosteronism.

21. A compound according to any one of claims 1 to 14 for use in a method for producing a diuretic effect.

22. A process for preparing a compound of Claim 1 which comprises reacting a compound of the formula 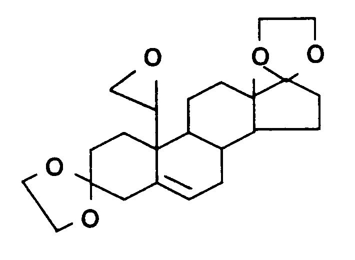 with a hydrohalic acid, i.e., hydrobromic, hydrochloric, or hydroiodic acid, in acetone to yield the compound of Claim 1.

23. A pharmaceutical composition comprising a compound according to any one of claims 1 to 14 and a pharmaceutically acceptable carrier.

## Claims (Claims for the following Contracting State(s): GR)

1. A compound of the formula: 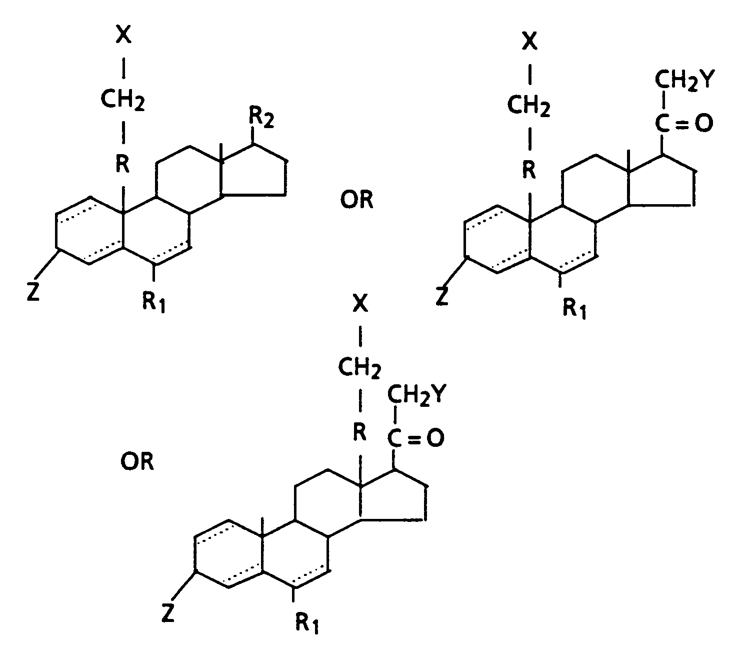 wherein
---- represents a single or double bond,
X = Br, Cl, or I,
R = CHOH or C=O,
R₁ = H, C₁₋₄ alkyl, =O, or -OH,
R₂ = =O, -OH, or -O-(C₁₋₄ alkanoyl),
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

2. A compound according to Claim 1 which has the formula 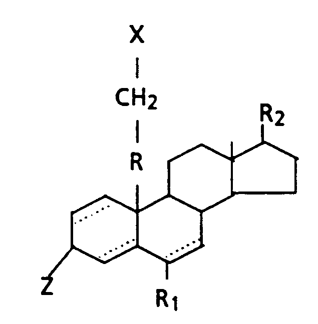 wherein
---- represents a single or double bond,
X = Br, Cl, or I,
R = CHOH,
R₁ = H, C₁₋₄ alkyl, =O, or -OH,
R₂ = =O, -OH, or -O-(C₁₋₄ alkanoyl), and
Z = =O, = CH₂, -OH, or -O-(C₁₋₄ alkanoyl)

3. A compound according to Claim 2 which has the formula 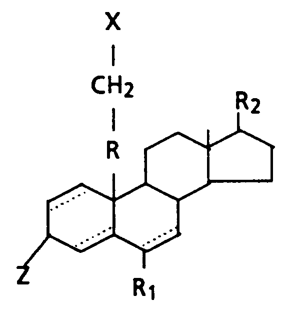 wherein
---- represents a single or double bond,
X = Br, Cl, or I,
R = CHOH,
R₁ = H or C₁₋₄ alkyl,
R₂ = =O or -OH, and
Z = =O, -OH, or -O-(C₁₋₄ alkanoyl).

4. A compound according to Claim 3 which has the formula 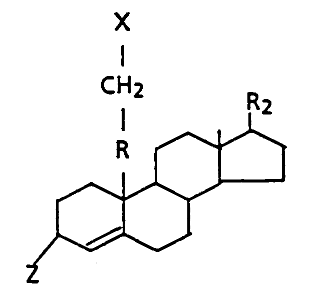 wherein
X = Br, Cl, or I,
R = CHOH,
R₂ = =O or -OH, and
Z = =O.

5. A compound according to Claim 4 which is 10-(2-bromo-1-hydroxyethyl)-estr-4-ene-3,17-dione.

6. A compound according to Claim 4 which is 10-(2-chloro-1-hydroxyethyl)-estr-4-ene-3,17-dione.

7. A compound according to Claim 4 which is 10-(2-iodo-1-hydroxyethyl)-estr-4-ene-3,17-dione.

8. A compound according to Claim 1 which is 10-(2-bromoacetyl)-estr-4-ene-3,17-dione.

9. A compound according to Claim 1 which has the formula 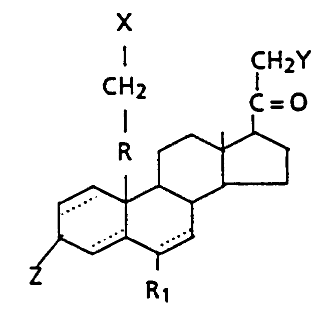 wherein
---- represents a single or double bond,
X = Br, Cl, or I,
R = CHOH or C=O,
R₁ = H, C₁₋₄ alkyl, =O, or -OH,
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

10. A compound according to Claim 9 which has the formula 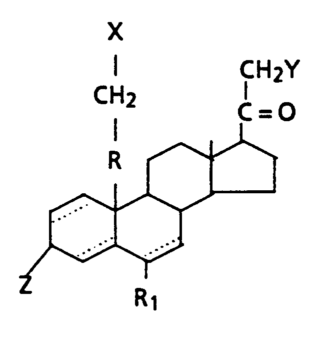 wherein
---- represents a single or double bond,
X = Br, Cl, or I,
R = CHOH,
R₁ = H or C₁₋₄ alkyl,
Z = =O, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH.

11. A compound of Claim 10 which has the formula 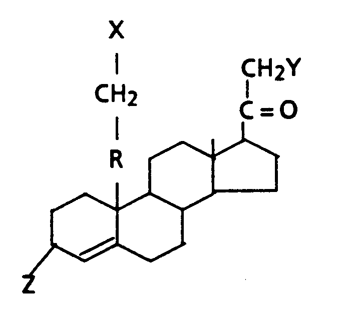 wherein
X = Br, Cl, or I,
R = CHOH,
Z = =0, and
Y = H, -OH, or -O-(C₁₋₄ alkanoyl).

12. A compound of Claim 1 which has the formula 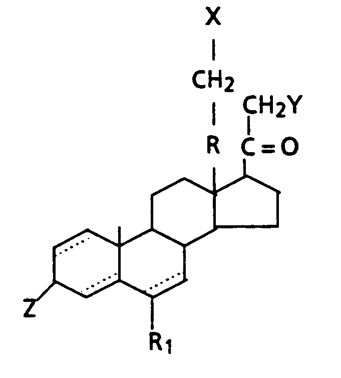 wherein
---- represents a single or double bond,
X = Br, Cl, or I,
R = CHOH or C=O,
R₁ = H, C₁₋₄ alkyl, =O, or -OH,
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH.

13. A compound according to Claim 12 which has the formula 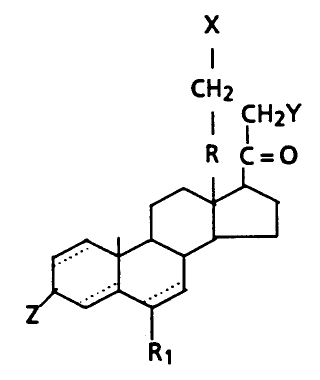 wherein
---- represents a single or double bond,
X = Br, Cl, or I,
R = CHOH,
R₁ = H or C₁₋₄ alkyl,
Z = =O, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH.

14. A compound of Claim 13 which has the formula 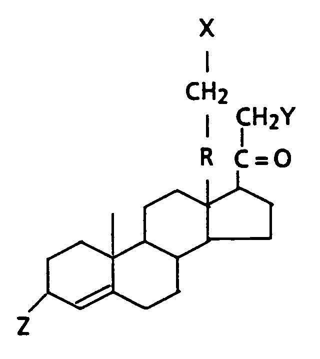 wherein
X = Br, Cl, or I,
R = CHOH,
Z = =O, and
Y = H, -OH, or -O-(C₁₋₄ alkanoyl).

15. A compound according to any one of claims 1 to 14 for use in a method of inhibiting aromatase activity, which comprises contacting an effective aromatase-inhibiting amount of the compound with an aromatase enzyme.

16. A compound according to any one of claims 1 to 14 for use in a method of treating hyperestrogenemia.

17. A compound according to any one of claims 1 to 14 for use in a method of treating estrogen-induced or estrogen-stimulated disorders.

18. A compound according to any one of claims 1 to 14 for use in a method in which the aromatase inhibitor produces an anti-fertility effect.

19. A compound according to any one of claims 1 to 14 for use in a method of treating hypertensive or edemous conditions.

20. A compound according to any one of claims 1 to 14 for use in a method for treating hyperaldosteronism.

21. A compound according to any one of claims 1 to 14 for use in a method for producing a diuretic effect.

22. A process for preparing a compound of Claim 1 which comprises reacting a compound of the formula 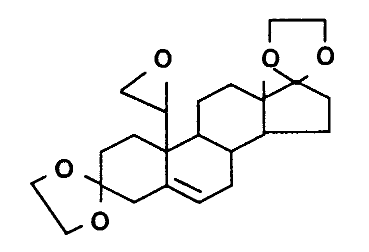 with a hydrohalic acid, i.e., hydrobromic, hydrochloric, or hydroiodic acid, in acetone to yield the compound of Claim 1.

## Claims (Claims for the following Contracting State(s): ES)

1. A process for preparing a compound of the formula: 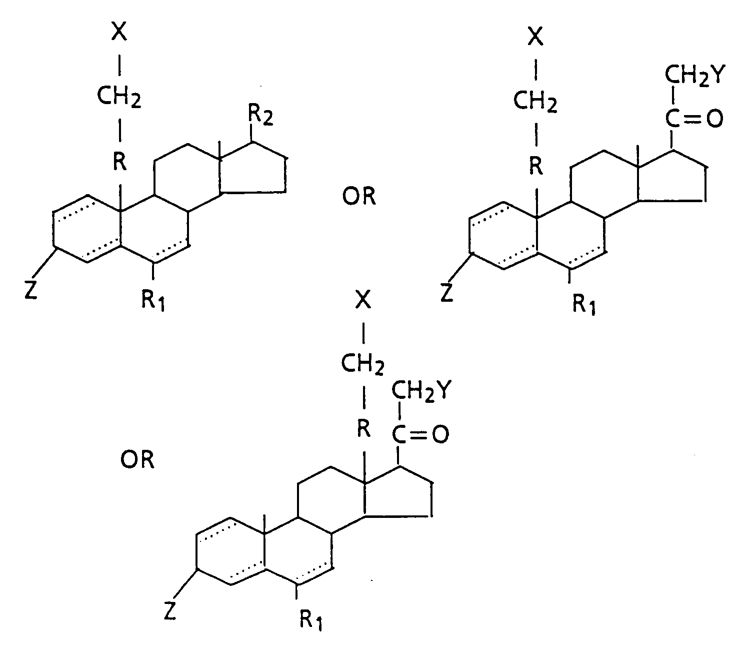 wherein
---- represents a single or double bond,
X = Br, Cl, or I,
R = CHOH or C=O,
R₁ = H, C₁₋₄ alkyl, =O, or -OH,
R₂ = =O, -OH, or -O-(C₁₋₄ alkanoyl),
Z = =O, =CH₂, -OH, or -O-(C₁₋₄ alkanoyl), and
Y = H, -OH, or -O-(C₁₋₄ alkanoyl), and when Y = H, -OH, or -O-(C₁₋₄ alkanoyl), Z may not include -OH, and R₁ may not include =O or -OH,
which comprises reacting the corresponding compound wherein X-CH₂-R- is 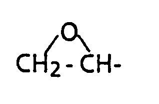 and any =O or -OH groups are optionally protected, with an acid of the formula HX, in an inert solvent, optionally followed by oxidation to give the compounds in which R is C=O.

2. A process according to Claim 1 for preparing a compound of the formula 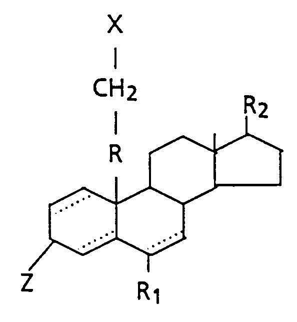 wherein
---- represents a single or double bond,
X = Br, Cl, or I,
R = CHOH or C=O,
R₁ = H, C₁₋₄ alkyl, =O, or -OH,
R₂ = =O, -OH, or -O-(C₁₋₄ alkanoyl), and
Z = =O, =CH₂, -OH, or -O-(C₁₋₄ alkanoyl),
which comprises reacting the corresponding compound wherein X-CH₂-R- is 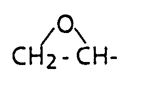 and any =O or -OH groups are optionally protected, with an acid of the formula HX, in an inert solvent, optionally followed by oxidation to give the compounds in which R is C=O.

3. A process according to Claim 1 for preparing a compound of the formula 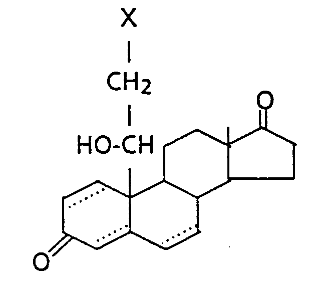 wherein
---- represents a single or double bond, and
X = Br, Cl, or I,
which comprises reacting an epoxide of the formula 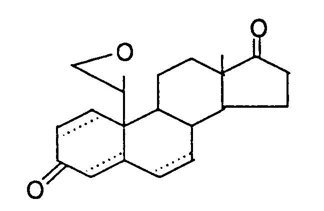 wherein the carbonyl groups are optionally protected, with an acid of the formula HX in an inert solvent.

4. A process according to Claim 1 for preparing 10-(2-chloro-1-hydroxyethyl)estr-4-ene-3,17-dione which comprises reacting 3,3,17,17-bis(ethylenedioxy)-10β₋oxiranylestr-5-ene with hydrochloric acid in an inert solvent.

5. A process according to Claim 1 for preparing 10-(2-bromo-1-hydroxyethyl)estr-4-ene-3,17-dione which comprises reacting 3,3,17,17-bis(ethylenedioxy)-10β-oxiranylestr-5-ene with hydrobromic acid in an inert solvent.

6. A process according to Claim 1 for preparing 10-(2-iodo-1-hydroxyethyl)estr-4-ene-3,17-dione which comprises reacting 3,3,17,17-bis(ethylenedioxy)-10β-oxiranylestr-5-ene with hydroiodic acid in an inert solvent.

## Patentansprüche (Patentansprüche für folgende(n) Vertragsstaat(en): AT, BE, CH, DE, DK, FR, GB IT, LI, LU, NL, SE)

1. Verbindung der Formel 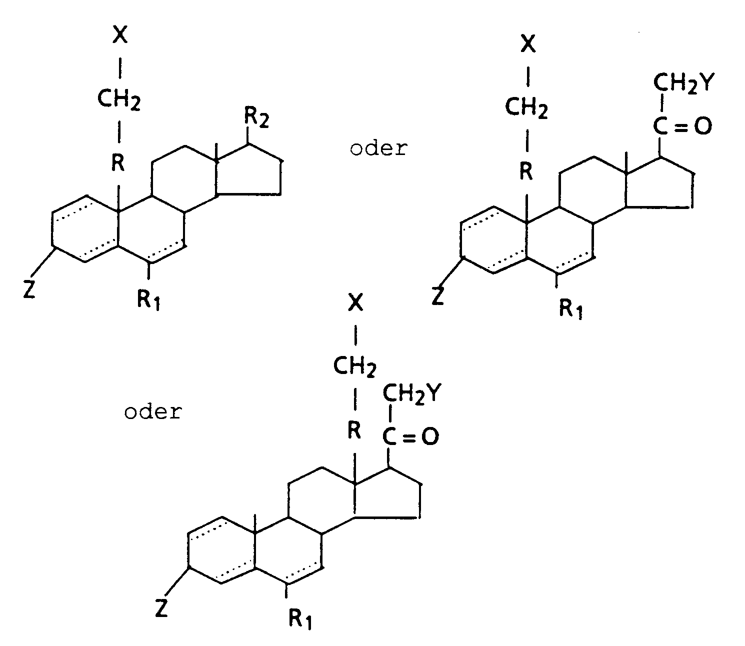 wobei
---- eine Einfach- oder Doppelbindung bedeutet,
X = Br, Cl oder I,
R = CHOH oder C=O,
R₁ = H, ein C₁₋₄-Alkylrest, =O oder -OH,
R₂ = =O, -OH oder ein -O-(C₁₋₄-Alkanoylrest),
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf und R₁ =O oder -OH nicht einschließen darf.

2. Verbindung nach Anspruch 1 mit der Formel 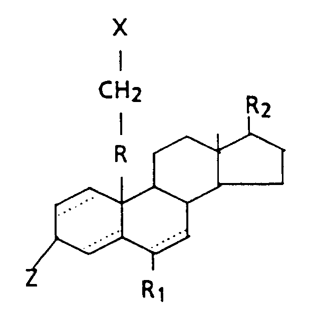 wobei
---- eine Einfach- oder Doppelbindung bedeutet,
X = Br, Cl oder I,
R = CHOH,
R₁ = H, ein C₁₋₄-Alkylrest, =O oder -OH,
R₂ = =O, -OH oder ein -O-(C₁₋₄-Alkanoylrest) und
Z = =O, =CH₂, -OH oder ein -O-(C₁₋₄-Alkanoylrest).

3. Verbindung nach Anspruch 2 mit der Formel 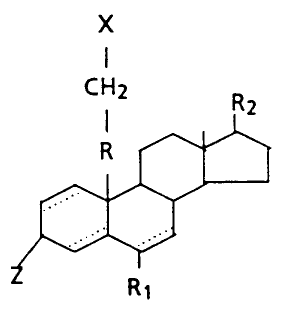 wobei
---- eine Einfach- oder Doppelbindung bedeutet,
X = Br, Cl oder I,
R = CHOH,
R₁ = H oder ein C₁₋₄-Alkylrest,
R₂ = =O oder -OH und
Z = =O, -OH oder ein -O-(C₁₋₄-Alkanoylrest).

4. Verbindung nach Anspruch 3 mit der Formel 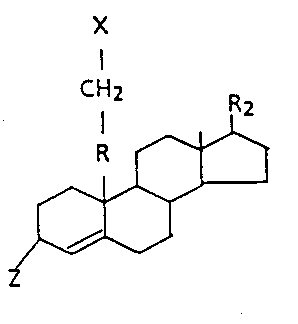 wobei
X = Br, Cl oder I,
R = CHOH,
R₂ = =O oder -OH und
Z = =O.

5. Verbindung nach Anspruch 4, die 10-(2-Brom-1-hydroxyethyl)-östr-4-en-3,17-dion ist.

6. Verbindung nach Anspruch 4, die 10-(2-Chlor-1-hydroxyethyl)-östr-4-en-3,17-dion ist.

7. Verbindung nach Anspruch 4, die 10-(2-Iod-1-hydroxyethyl)-östr-4-en-3,17-dion ist.

8. Verbindung nach Anspruch 1, die 10-(2-Bromacetyl)-östr-4-en-3,17-dion ist.

9. Verbindung nach Anspruch 1 mit der Formel 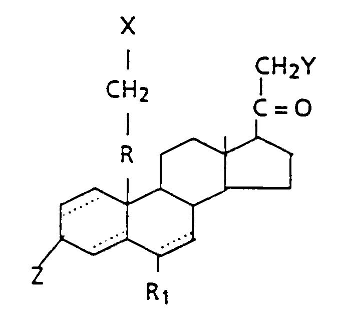 wobei
---- eine Einfach- oder Doppelbindung bedeutet,
X = Br, Cl oder I,
R = CHOH oder C=O,
R₁ = H, ein C₁₋₄-Alkylrest, =O oder -OH,
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf und R₁ =O oder -OH nicht einschließen darf.

10. Verbindung nach Anspruch 9 mit der Formel 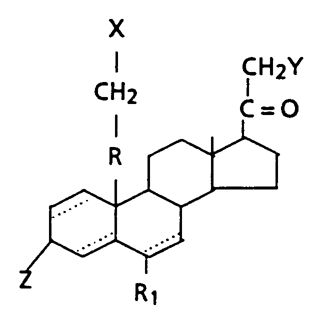 wobei
---- eine Einfach- oder Doppelbindung bedeutet,
X = Br, Cl oder I,
R = CHOH,
R₁ = H oder ein C₁₋₄-Alkylrest,
Z = =O, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf.

11. Verbindung nach Anspruch 10 mit der Formel 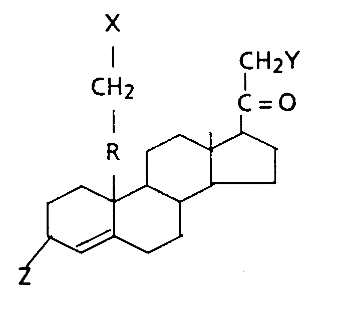 wobei
X = Br, Cl oder I,
R = CHOH,
Z = =O und
Y = H, -OH oder ein -O-(C₁₋₄-Alkanoylrest).

12. Verbindung nach Anspruch 1 mit der Formel 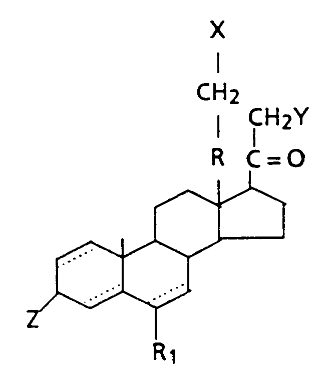 wobei
---- eine Einfach- oder Doppelbindung bedeutet,
X = Br, Cl oder I,
R = CHOH oder C=O,
R₁ = H, ein C₁₋₄-Alkylrest, =O oder -OH,
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest),Z -OH nicht einschließen darf und R₁ =O oder -OH nicht einschließen darf.

13. Verbindung nach Anspruch 12 mit der Formel 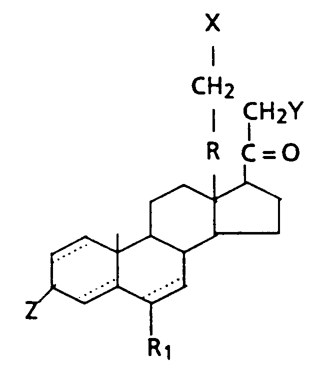 wobei
---- eine Einfach- oder Doppelbindung bedeutet,
X = Br, Cl oder I,
R = CHOH,
R₁ = H oder ein C₁₋₄-Alkylrest,
Z = =O, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf.

14. Verbindung nach Anspruch 13 mit der Formel 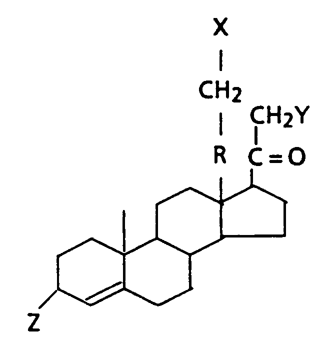 wobei
X = Br, Cl oder I,
R = CHOH,
Z = =O und
Y = H, -OH oder ein -O-(C₁₋₄-Alkanoylrest).

15. Verbindung nach einem der Ansprüche 1 bis 14 zur Verwendung in einem Verfahren zur Hemmung der Aromataseaktivität, umfassend das Inkontaktbringen einer wirksamen Aromatase hemmenden Menge der Verbindung mit einem Aromataseenzym.

16. Verbindung nach einem der Ansprüche 1 bis 14 zur Verwendung in einem Verfahren zur Behandlung von Hyperöstrogenämie.

17. Verbindung nach einem der Ansprüche 1 bis 14 zur Verwendung in einem Verfahren zur Behandlung durch Östrogen verursachter oder durch Östrogen verstärkter Erkrankungen.

18. Verbindung nach einem der Ansprüche 1 bis 14 zur Verwendung in einem Verfahren, in dem der Aromataseinhibitor eine fertilitätshemmende Wirkung hervorruft.

19. Verbindung nach einem der Ansprüche 1 bis 14 zur Verwendung in einem Verfahren zur Behandlung hypertoner oder ödematöser Erkrankungen.

20. Verbindung nach einem der Ansprüche 1 bis 14 zur Verwendung in einem Verfahren zur Behandlung von Hyperaldosteronismus.

21. Verbindung nach einem der Ansprüche 1 bis 14 zur Verwendung in einem Verfahren, das eine diuretische Wirkung hervorruft.

22. Verfahren zur Herstellung einer Verbindung nach Anspruch 1, umfassend die Umsetzung einer Verbindung der Formel 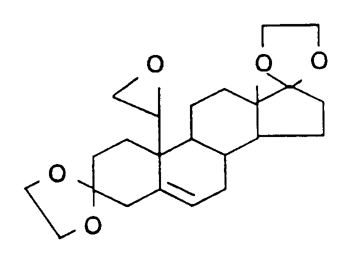 mit einer Halogenwasserstoffsäure, d.h. Bromwasserstoffsäure, Salzsäure oder Iodwasserstoffsäure, in Aceton, wobei die Verbindung nach Anspruch 1 erhalten wird.

23. Arzneimittel, umfassend eine Verbindung nach einem der Ansprüche 1 bis 14 und einen pharmazeutisch verträglichen Träger.

## Patentansprüche (Patentansprüche für folgende(n) Vertragsstaat(en): GR)

1. Verbindung der Formel 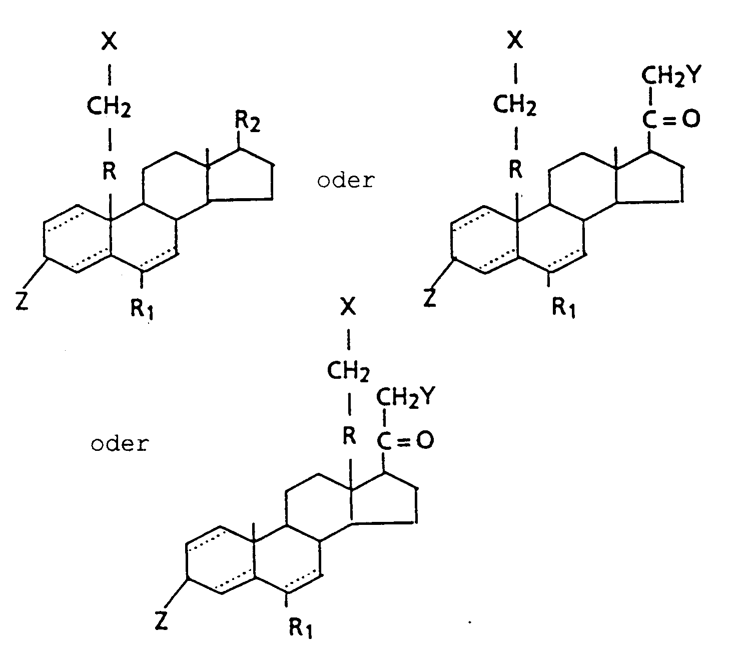 wobei
---- eine Einfach- oder Doppelbindung bedeutet,
X = Br, Cl oder I,
R = CHOH oder C=O,
R₁ = H, ein C₁₋₄-Alkylrest, =O oder -OH,
R₂ = =O, -OH oder ein -O-(C₁₋₄-Alkanoylrest),
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf und R₁ =O oder -OH nicht einschließen darf.

2. Verbindung nach Anspruch 1 mit der Formel 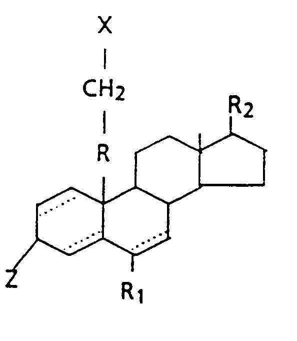 wobei
---- eine Einfach- oder Doppelbindung bedeutet,
X = Br, Cl oder I,
R = CHOH,
R₁ = H, ein C₁₋₄-Alkylrest, =O oder -OH,
R₂ = =O, -OH oder ein -O-(C₁₋₄-Alkanoylrest) und
Z = =O, =CH₂, -OH oder ein -O-(C₁₋₄-Alkanoylrest).

3. Verbindung nach Anspruch 2 mit der Formel 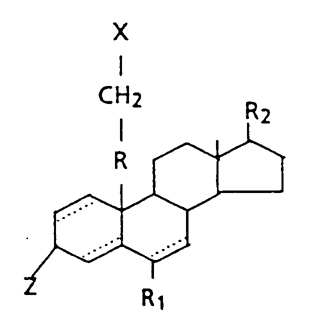 wobei
---- eine Einfach- oder Doppelbindung bedeutet,
X = Br, Cl oder I,
R = CHOH,
R₁ = H oder ein C₁₋₄-Alkylrest,
R₂ = =O oder -OH und
Z = =O, -OH oder ein -O-(C₁₋₄-Alkanoylrest).

4. Verbindung nach Anspruch 3 mit der Formel 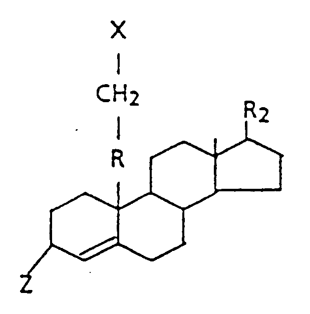 wobei
X = Br, Cl oder I,
R = CHOH,
R₂ = =O oder -OH und
Z = =O.

5. Verbindung nach Anspruch 4, die 10-(2-Brom-1-hydroxyethyl)-östr-4-en-3,17-dion ist.

6. Verbindung nach Anspruch 4, die 10-(2-Chlor-1-hydroxyethyl)-östr-4-en-3,17-dion ist.

7. Verbindung nach Anspruch 4, die 10-(2-Iod-1-hydroxyethyl)-östr-4-en-3,17-dion ist.

8. Verbindung nach Anspruch 1, die 10-(2-Bromacetyl)-östr-4-en-3,17-dion ist.

9. Verbindung nach Anspruch 1 mit der Formel 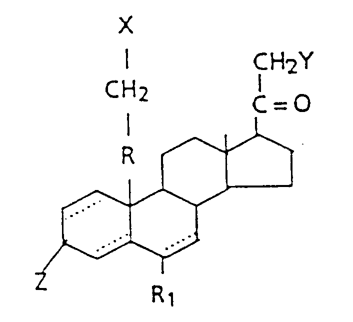 wobei
---- eine Einfach- oder Doppelbindung bedeutet,
X = Br, Cl oder I,
R = CHOH oder C=O,
R₁ = H, ein C₁₋₄-Alkylrest, =O oder -OH,
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf und R₁ =O oder -OH nicht einschließen darf.

10. Verbindung nach Anspruch 9 mit der Formel 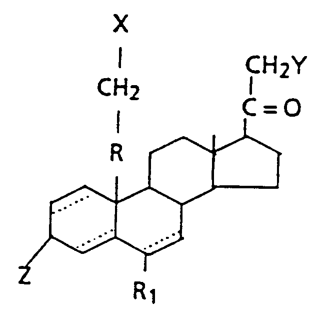 wobei
---- eine Einfach- oder Doppelbindung bedeutet,
X = Br, Cl oder I,
R = CHOH,
R₁ = H oder ein C₁₋₄-Alkylrest,
Z = =O, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf.

11. Verbindung nach Anspruch 10 mit der Formel 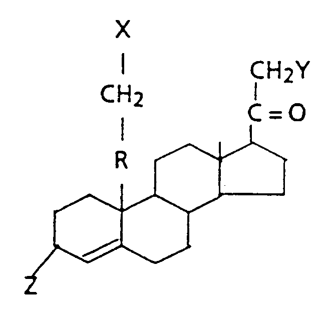 wobei
X = Br, Cl oder I,
R = CHOH,
Z = =O und
Y = H, -OH oder ein -O-(C₁₋₄-Alkanoylrest).

12. Verbindung nach Anspruch 1 mit der Formel 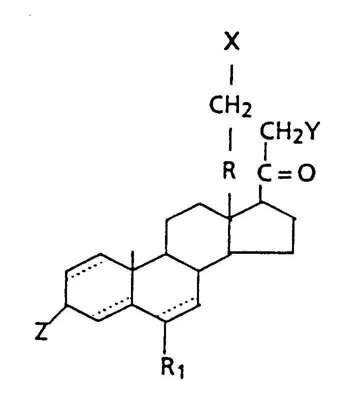 wobei
---- eine Einfach- oder Doppelbindung bedeutet,
X = Br, Cl oder I,
R = CHOH oder C=O,
R₁ = H, ein C₁₋₄-Alkylrest, =O oder -OH,
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest),Z -OH nicht einschließen darf und R₁ =O oder -OH nicht einschließen darf.

13. Verbindung nach Anspruch 12 mit der Formel 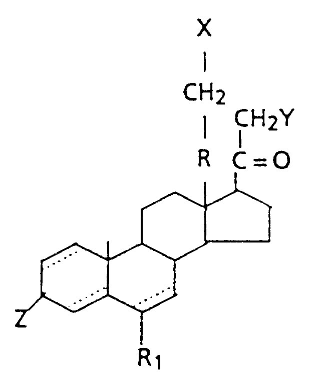 wobei
---- eine Einfach- oder Doppelbindung bedeutet,
X = Br, Cl oder I,
R = CHOH,
R₁ = H oder ein C₁₋₄-Alkylrest,
Z = =O, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf.

14. Verbindung nach Anspruch 13 mit der Formel 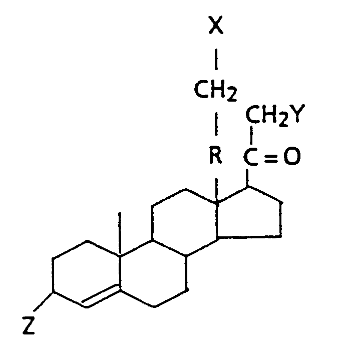 wobei
X = Br, Cl oder I,
R = CHOH,
Z = =O und
Y = H, -OH oder ein -O-(C₁₋₄-Alkanoylrest).

15. Verbindung nach einem der Ansprüche 1 bis 14 zur Verwendung in einem Verfahren zur Hemmung der Aromataseaktivität, umfassend das Inkontaktbringen einer wirksamen Aromatase hemmenden Menge der Verbindung mit einem Aromataseenzym.

16. Verbindung nach einem der Ansprüche 1 bis 14 zur Verwendung in einem Verfahren zur Behandlung von Hyperöstrogenämie.

17. Verbindung nach einem der Ansprüche 1 bis 14 zur Verwendung in einem Verfahren zur Behandlung durch Östrogen verursachter oder durch Östrogen verstärkter Erkrankungen.

18. Verbindung nach einem der Ansprüche 1 bis 14 zur Verwendung in einem Verfahren, in dem der Aromataseinhibitor eine fertilitätshemmende Wirkung hervorruft.

19. Verbindung nach einem der Ansprüche 1 bis 14 zur Verwendung in einem Verfahren zur Behandlung hypertoner oder ödematöser Erkrankungen.

20. Verbindung nach einem der Ansprüche 1 bis 14 zur Verwendung in einem Verfahren zur Behandlung von Hyperaldosteronismus.

21. Verbindung nach einem der Ansprüche 1 bis 14 zur Verwendung in einem Verfahren, das eine diuretische Wirkung hervorruft.

22. Verfahren zur Herstellung einer Verbindung nach Anspruch 1, umfassend die Umsetzung einer Verbindung der Formel 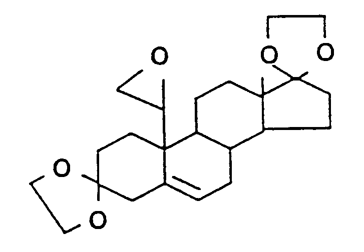 mit einer Halogenwasserstoffsäure, d.h. Bromwasserstoffsäure, Salzsäure oder Iodwasserstoffsäure, in Aceton, wobei die Verbindung nach Anspruch 1 erhalten wird.

## Patentansprüche (Patentansprüche für folgende(n) Vertragsstaat(en): ES)

1. Verfahren zur Herstellung einer Verbindung der Formel 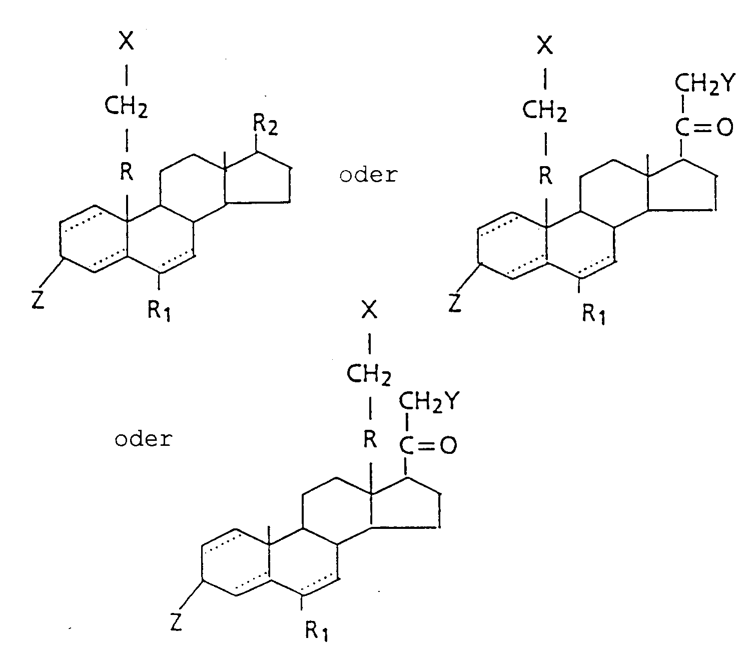 wobei
---- eine Einfach- oder Doppelbindung bedeutet,
X = Br, Cl oder I,
R = CHOH oder C=O,
R₁ = H, ein C₁₋₄-Alkylrest, =O oder -OH,
R₂ = =O, -OH oder ein -O-(C₁₋₄-Alkanoylrest),
Z = =O, =CH₂, -OH oder ein -O-(C₁₋₄-Alkanoylrest) und
Y = H, -OH oder ein -O-(C₁₋₄-Alkanoylrest), wobei wenn Y = H, -OH oder ein -O-(C₁₋₄-Alkanoylrest), Z -OH nicht einschließen darf und R₁ =O oder -OH nicht einschließen darf,
umfassend die Umsetzung der entsprechenden Verbindung, in der X-CH₂-R- 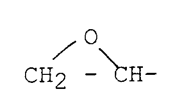 bedeutet und jede Gruppe =O oder -OH gegebenenfalls geschützt ist, mit einer Säure der Formel HX in einem inerten Lösungsmittel, gegebenenfalls gefolgt von einer Oxidation, wobei die Verbindungen erhalten werden, in denen R C=O bedeutet.

2. Verfahren nach Anspruch 1, zur Herstellung einer Verbindung der Formel 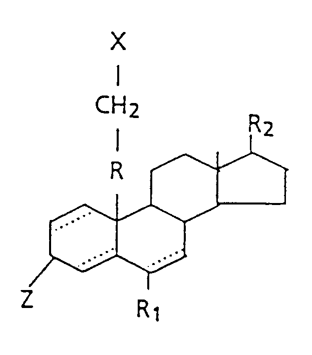 wobei
---- eine Einfach- oder Doppelbindung bedeutet,
X = Br, Cl oder I,
R = CHOH oder C=O,
R₁ = H, ein C₁₋₄-Alkylrest, =O oder -OH,
R₂ = =O, -OH oder ein -O-(C₁₋₄-Alkanoylrest) und
Z = =O, =CH₂, -OH oder ein -O-(C₁₋₄-Alkanoylrest), umfassend die Umsetzung der entsprechenden Verbindung, in der X-CH₂-R- 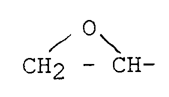 bedeutet und jede Gruppe =O oder -OH gegebenenfalls geschützt ist, mit einer Säure der Formel HX in einem inerten Lösungsmittel, gegebenenfalls gefolgt von einer Oxidation, wobei die Verbindungen erhalten werden, in denen R C=O bedeutet.

3. Verfahren nach Anspruch 1 zur Herstellung einer Verbindung der Formel 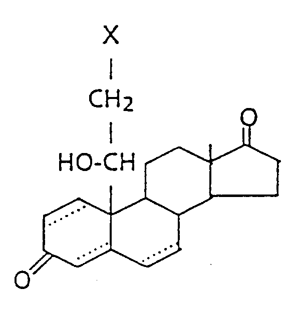 wobei
---- eine Einfach- oder Doppelbindung bedeutet und
X = Br, Cl oder I,
umfassend die Umsetzung eines Epoxids der Formel 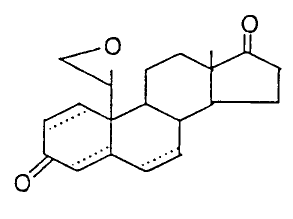 wobei die Carbonylgruppen gegebenenfalls geschützt sind, mit einer Säure der Formel HX in einem inerten Lösungsmittel.

4. Verfahren nach Anspruch 1 zur Herstellung von 10-(2-Chlor-1-hydroxyethyl)-östr-4-en-3,17-dion, umfassend die Umsetzung von 3,3,17,17-Bis(ethylendioxy)-10β-oxiranyl-östr-5-en mit Salzsäure in einem inerten Lösungsmittel.

5. Verfahren nach Anspruch 1 zur Herstellung von 10-(2-Brom-1-hydroxyethyl)-östr-4-en-3,17-dion, umfassend die Umsetzung von 3,3,17,17-Bis(ethylendioxy)-10β-oxiranyl-östr-5-en mit Bromwasserstoffsäure in einem inerten Lösungsmittel.

6. Verfahren nach Anspruch 1 zur Herstellung von 10-(2-Iod-1-hydroxyethyl)-östr-4-en-3,17-dion, umfassend die Umsetzung von 3,3,17,17-Bis(ethylendioxy)-10β-oxiranylöstr-5-en mit Jodwasserstoffsäure in einem inerten Lösungsmittel.

## Revendications (Revendications pour l'(les) Etat(s) contractant(s) suivant(s): AT, BE, CH, DE, DK, FR, GB, IT, LI, LU, NL, SE)

1. Un composé de formule : 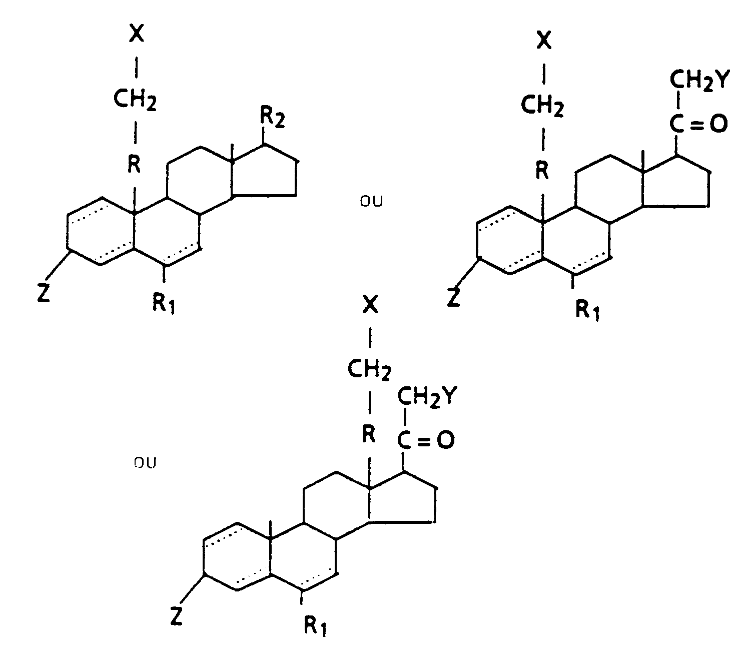 dans laquelle
---- représente une liaison simple ou double,
X = Br, Cl ou I,
R = CHOH ou C=O,
R₁ = H, C₁-C₄ alkyle, =O ou -OH,
R₂ = =O, -OH, ou -O-(C₁-C₄ alcanoyle),
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

2. Un composé selon la revendication 1 qui a la formule 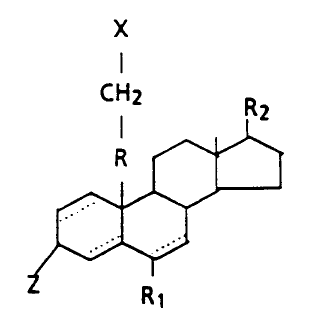 dans laquelle
---- représente une liaison simple ou double,
X = Br, Cl ou I,
R = CHOH,
R₁ = H, C₁-C₄ alkyle, =O ou -OH,
R₂ = =O, -OH, ou -O-(C₁-C₄ alcanoyle), et
Z = =O, =CH₂, -OH, ou -O-(C₁-C₄ alcanoyle)

3. Un composé selon la revendication 2 qui a la formule 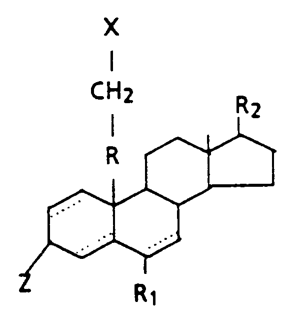 dans laquelle
---- représente une liaison simple ou double,
X = Br, Cl ou I,
R = CHOH,
R₁ = H ou C₁-C₄ alkyle,
R₂ = =O ou -OH, et
Z = =O, -OH, ou -O-(C₁-C₄ alcanoyle).

4. Un composé selon la revendication 3 qui a la formule 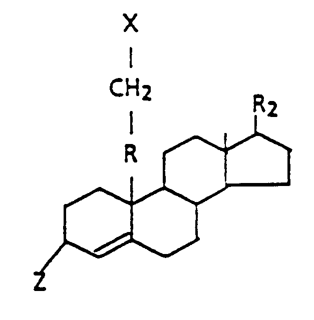 dans laquelle
X = Br, Cl ou I,
R = CHOH,
R₂ = =O ou -OH, et
Z = =O.

5. Un composé selon la revendication 4 qui est la 10-(2-bromo-1-hydroxyéthyl)-oestra-4-ène-3,17-dione.

6. Un composé selon la revendication 4 qui est la 10-(2-chloro-1-hydroxyéthyl)-oestra-4-ène-3,17-dione.

7. Un composé selon la revendication 4 qui est la 10-(2-iodo-1-hydroxyéthyl)-oestra-4-ène-3,17-dione.

8. Un composé selon la revendication 1 qui est la 10-(2-bromo-acétyl)-oestra-4-ène-3,17-dione.

9. Un composé selon la revendication 1 qui a la formule 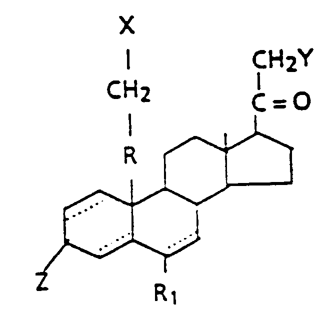 dans laquelle
---- représente une liaison simple ou double,
X = Br, Cl ou I,
R = CHOH ou C=O,
R₁ = H, C₁-C₄ alkyle, =O ou -OH,
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

10. Un composé selon la revendication 9 qui a la formule 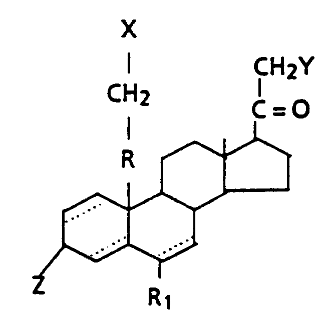 dans laquelle
---- représente une liaison simple ou double,
X = Br, Cl ou I,
R = CHOH,
R₁ = H ou C₁-C₄ alkyle,
Z = =O, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH.

11. Composé selon la revendication 10 qui a la formule 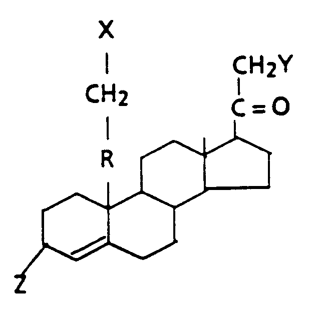 dans laquelle
X = Br, Cl ou I,
R = CHOH,
Z = =O, et
Y = H, -OH, ou -O-(C₁-C₄ alcanoyle).

12. Un composé selon la revendication 1 qui a la formule 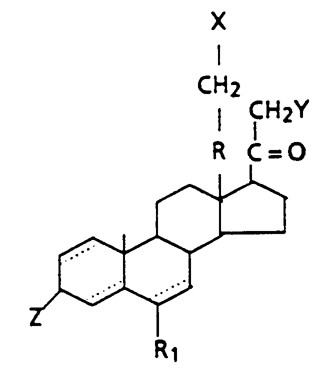 dans laquelle
---- représente une liaison simple ou double,
X = Br, Cl ou I,
R = CHOH ou C=O,
R₁ = H, C₁-C₄ alkyle, =O ou -OH,
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

13. Un composé selon la revendication 12 qui a la formule 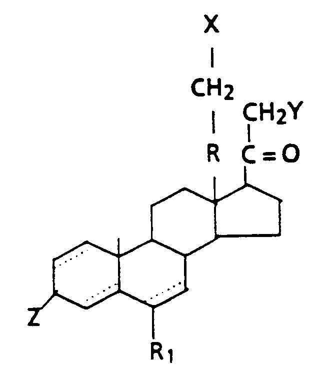 dans laquelle
---- représente une liaison simple ou double,
X = Br, Cl ou I,
R = CHOH,
R₁ = H ou alkyle en C₁-C₄,
Z = =O, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH.

14. Un composé selon la revendication 13 qui a la formule 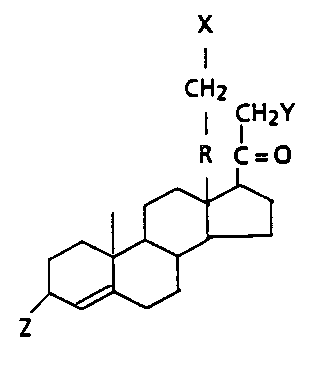 dans laquelle
X = Br, Cl ou I,
R = CHOH,
Z = =O, et
Y = H, -OH, ou -O-(C₁-C₄ alcanoyle).

15. Un composé selon l'une quelconque des revendications 1 à 14 à utiliser dans une méthode d'inhibition de l'activité de l'aromatase, qui comprend la mise en contact d'une quantité inhibitrice d'aromatase efficace du composé avec une enzyme aromatase.

16. Un composé selon l'une quelconque des revendications 1 à 14 à utiliser dans une méthode de traitement d'hyperoestrogénémie.

17. Un composé selon l'une quelconque des revendications 1 à 14 à utiliser dans une méthode de traitement de troubles induits par un oestrogène ou stimulés par un oestrogène.

18. Un composé selon l'une quelconque des revendications 1 à 14 à utiliser dans une méthode dans laquelle l'inhibiteur de l'aromatase produit un effet antifécondité.

19. Un composé selon l'une quelconque des revendications 1 à 14 à utiliser dans une méthode de traitement des états hypertensifs ou oedémateux.

20. Un composé selon l'une quelconque des revendications 1 à 14 à utiliser dans une méthode pour le traitement de l'hyperaldostéronisme.

21. Un composé selon l'une quelconque des revendications 1 à 14 à utiliser dans une méthode de production d'un effet diurétique.

22. Un procédé de préparation d'un composé selon la revendication 1 qui comprend la réaction d'un composé de formule 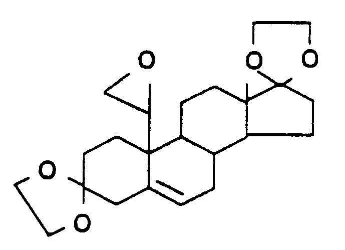 avec un acide halohalique, par exemple l'acide bromhydrique, chlorhydrique, ou iodhydrique, dans l'acétone pour donner le composé de la revendication 1.

23. Une composition pharmaceutique comprenant un composé selon l'une quelconque des revendications 1 à 14 et un support pharmaceutiquement acceptable.

## Revendications (Revendications pour l'(les) Etat(s) contractant(s) suivant(s): GR)

1. Un composé de formule : 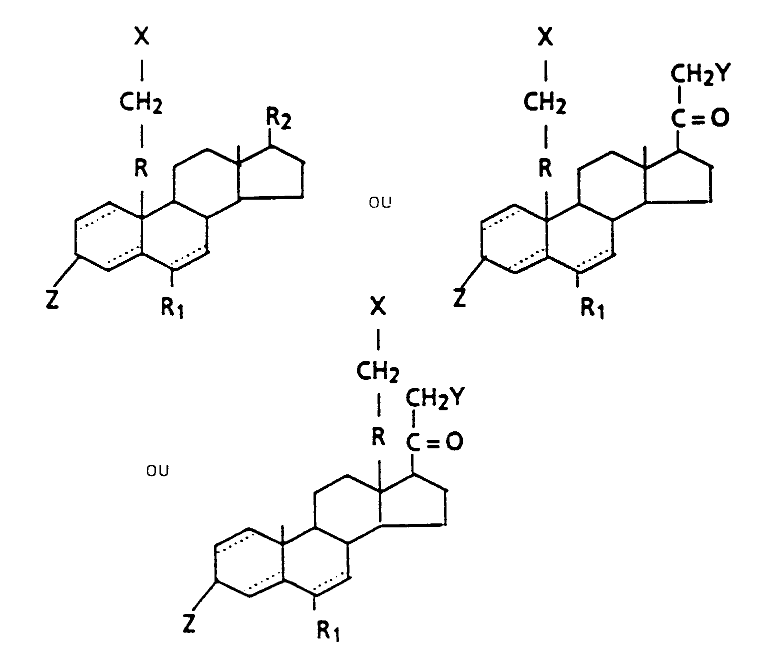 dans laquelle
---- représente une liaison simple ou double,
X = Br, Cl ou I,
R = CHOH ou C=O,
R₁ = H, C₁-C₄ alkyle, =O ou -OH,
R₂ = =O, -OH, ou -O-(C₁-C₄ alcanoyle),
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

2. Un composé selon la revendication 1 qui a la formule 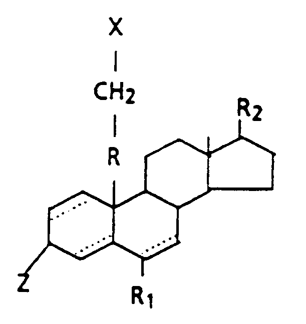 dans laquelle
---- représente une liaison simple ou double,
X = Br, Cl ou I,
R = CHOH,
R₁ = H, C₁-C₄ alkyle, =O ou -OH,
R₂ = =O, -OH, ou -O-(C₁-C₄ alcanoyle), et
Z = =O, =CH₂, -OH, ou -O-(C₁-C₄ alcanoyle)

3. Un composé selon la revendication 2 qui a la formule 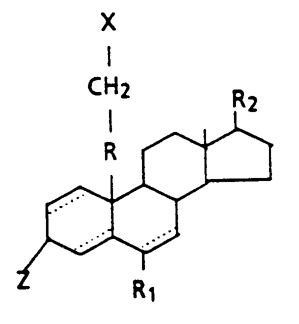 dans laquelle
---- représente une liaison simple ou double,
X = Br, Cl ou I,
R = CHOH,
R₁ = H ou C₁-C₄ alkyle,
R₂ = =O ou -OH, et
Z = =O, -OH, ou -O-(C₁-C₄ alcanoyle).

4. Un composé selon la revendication 3 qui a la formule 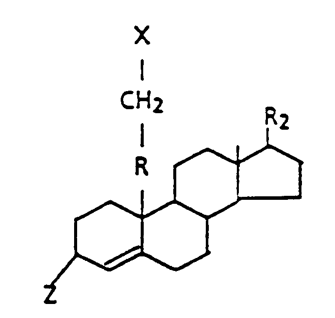 dans laquelle
X = Br, Cl ou I,
R = CHOH,
R₂ = =O ou -OH, et
Z = =O.

5. Un composé selon la revendication 4 qui est la 10-(2-bromo-1-hydroxyéthyl)-oestra-4-ène-3,17-dione.

6. Un composé selon la revendication 4 qui est la 10-(2-chloro-1-hydroxyéthyl)-oestra-4-ène-3,17-dione.

7. Un composé selon la revendication 4 qui est la 10-(2-iodo-1-hydroxyéthyl)-oestra-4-ène-3,17-dione.

8. Un composé selon la revendication 1 qui est la 10-(2-bromo-acétyl)-oestra-4-ène-3,17-dione.

9. Un composé selon la revendication 1 qui a la formule 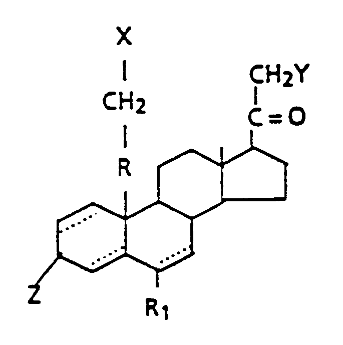 dans laquelle
---- représente une liaison simple ou double,
X = Br, Cl ou I,
R = CHOH ou C=O,
R₁ = H, C₁-C₄ alkyle, =O ou -OH,
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

10. Un composé selon la revendication 9 qui a la formule 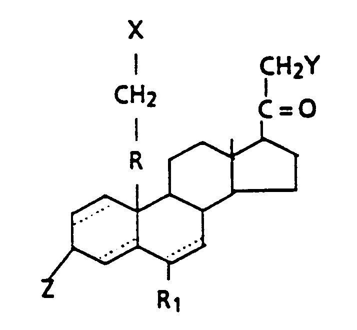 dans laquelle
---- représente une liaison simple ou double,
X = Br, Cl ou I,
R = CHOH,
R₁ = H ou C₁-C₄ alkyle,
Z = =O, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH.

11. Composé selon la revendication 10 qui a la formule 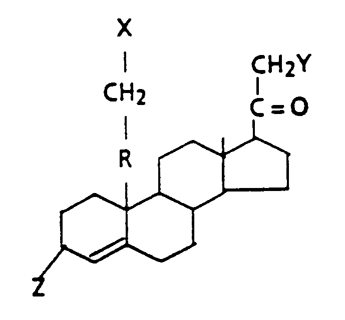 dans laquelle
X = Br, Cl ou I,
R = CHOH,
Z = =O, et
Y = H, -OH, ou -O-(C₁-C₄ alcanoyle).

12. Un composé selon la revendication 1 qui a la formule 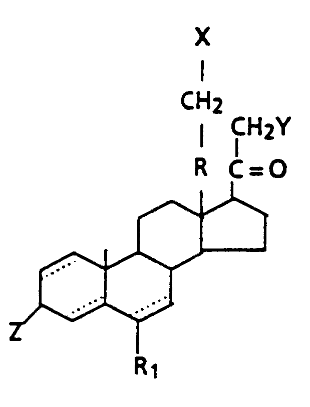 dans laquelle
---- représente une liaison simple ou double,
X = Br, Cl ou I,
R = CHOH ou C=O,
R₁ = H, C₁-C₄ alkyle, =O ou -OH,
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.

13. Un composé selon la revendication 12 qui a la formule 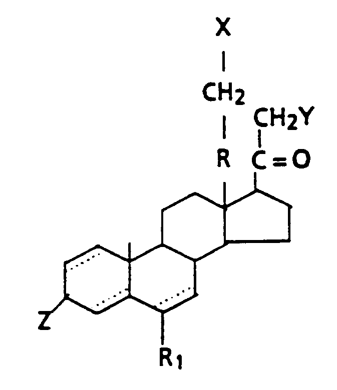 dans laquelle
---- représente une liaison simple ou double,
X = Br, Cl ou I,
R = CHOH,
R₁ = H ou C₁-C₄ alkyle,
Z = =O, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH.

14. Un composé selon la revendication 13 qui a la formule 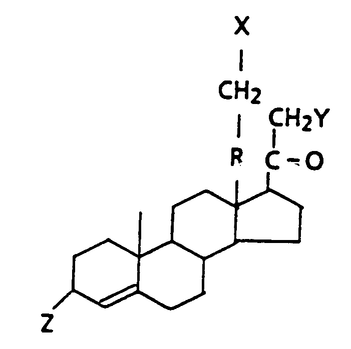 dans laquelle
X = Br, Cl ou I,
R = CHOH,
Z = =O, et
Y = H, -OH, ou -O-(C₁-C₄ alcanoyle).

15. Un composé selon l'une quelconque des revendications 1 à 14 à utiliser dans une méthode d'inhibition de l'activité de l'aromatase, qui comprend la mise en contact d'une quantité inhibitrice d'aromatase efficace du composé avec une enzyme aromatase.

16. Un composé selon l'une quelconque des revendications 1 à 14 à utiliser dans une méthode de traitement d'hyperoestrogénémie.

17. Un composé selon l'une quelconque des revendications 1 à 14 à utiliser dans une méthode de traitement de troubles induits par un oestrogène ou stimulés par un oestrogène.

18. Un composé selon l'une quelconque des revendications 1 à 14 à utiliser dans une méthode dans laquelle l'inhibiteur de l'aromatase produit un effet antifécondité.

19. Un composé selon l'une quelconque des revendications 1 à 14 à utiliser dans une méthode de traitement des états hypertensifs ou oedémateux.

20. Un composé selon l'une quelconque des revendications 1 à 14 à utiliser dans une méthode pour le traitement de l'hyperaldostéronisme.

21. Un composé selon l'une quelconque des revendications 1 à 14 à utiliser dans une méthode de production d'un effet diurétique.

22. Un procédé de préparation d'un composé selon la revendication 1 qui comprend la réaction d'un composé de formule 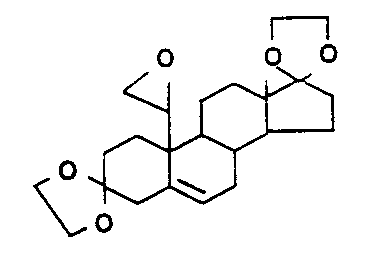 avec un acide halohalique, par exemple l'acide bromhydrique, chlorhydrique, ou iodhydrique, dans l'acétone pour donner le composé de la revendication 1.

## Revendications (Revendications pour l'(les) Etat(s) contractant(s) suivant(s): ES)

1. Un procédé de préparation d'un composé de formule : 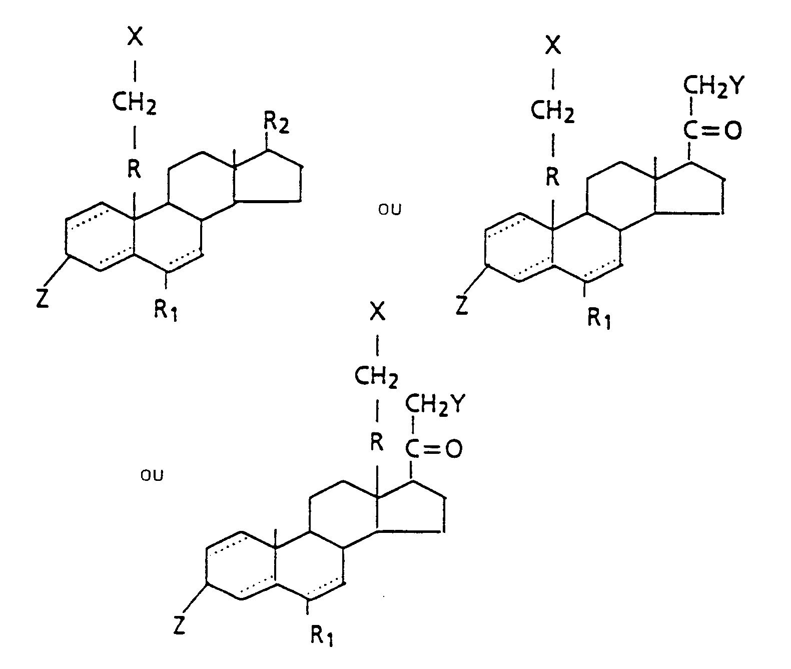 dans laquelle
---- représente une liaison simple ou double,
X = Br, Cl ou I,
R = CHOH ou C=O,
R₁ = H, C₁-C₄ alkyle, =O ou -OH,
R₂ = =O, -OH, ou -O-(C₁-C₄ alcanoyle),
Z = =O, =CH₂, -OH, ou -O-(C₁-C₄ alcanoyle), et
Y = H, -OH, ou -O-(C₁-C₄ alcanoyle), et lorsque Y = H, -OH, ou -O-(C₁-C₄ alcanoyle), Z peut ne pas inclure -OH et R₁ peut ne pas inclure =O ou -OH.
qui comprend la réaction du composé correspondant dans lequel X-CH₂-R- est 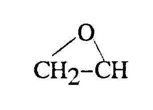 et les groupes =O ou -OH sont éventuellement protégés, avec un acide de formule HX dans un solvant inerte, éventuellement suivie d'une oxydation pour donner les composés dans lesquels R est C=O.

2. Un procédé selon la revendication 1 pour la préparation d'un composé de formule 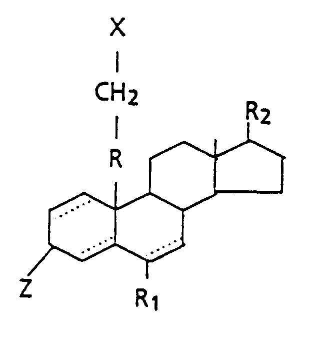 dans laquelle
---- représente une liaison simple ou double,
X = Br, Cl ou I,
R = CHOH ou C=O,
R₁ = H, C₁-C₄ alkyle, =O ou -OH,
R₂ = =O, -OH, ou -O-(C₁-C₄ alcanoyle), et
Z = =O, =CH₂, -OH, ou -O-(C₁-C₄ alcanoyle),
qui comprend la réaction du composé correspondant dans lequel
X-CH₂-R- est 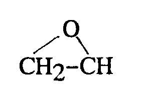 et les groupes =O ou -OH sont éventuellement protégés, avec un acide de formule HX, dans un solvant inerte, éventuellement, suivie d'une oxydation pour donner les composés dans lesquels R est C=O.

3. Un procédé selon la revendication 1 pour la préparation d'un composé de formule 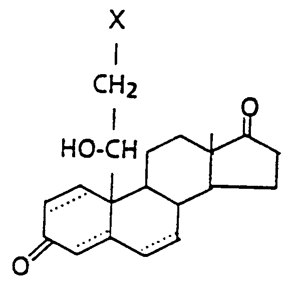 dans laquelle
---- représente une liaison simple ou double,
X = Br, Cl ou I,
qui comprend la réaction d'un époxyde de formule 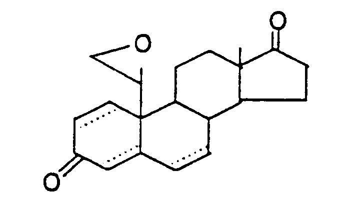 dans laquelle les groupes carbonyles sont éventuellement protégés, avec un acide de formule HX dans un solvant inerte.

4. Un procédé selon la revendication 1 pour la préparation de la 10-(2-chloro-1-hydroxyéthyl)oestra-4-ène-3,17-dione qui comprend la réaction du 3,3,17,17-bis(éthylènedioxy)-10β-oxirannyloestra-5-ène avec l'acide chlorhydrique dans un solvant inerte.

5. Un procédé selon la revendication 1 pour la préparation de la 10-(2-bromo-1-hydroxyéthyl)oestra-4-ène-3,17-dione qui comprend la réaction du 3,3,17,17-bis(éthylènedioxy)-10β-oxirannyloestra-5-ène avec l'acide bromhydrique dans un solvant inerte.

6. Un procédé selon la revendication 1 pour la préparation de la 10-(2-iodo-1-hydroxyéthyl)oestra-4-ène-3,17-dione qui comprend la réaction du 3,3,17,17-bis(éthylènedioxy)-10β-oxirannyloestra-5-ène avec l'acide iodhydrique dans un solvant inerte.
